# EUROPÄISCHE PATENTANMELDUNG

(11) **EP 1 980 202 A1**
(43) Veröffentlichungstag der Anmeldung: **15.10.2008**
(21) Anmeldenummer: 06851093.2
(22) Anmeldetag: 18.10.2006
(51) Int. Cl.: A61B 5/107

(54) **TRAGBARE AUTONOME DIGITALEINHEIT ZUR DURCHFÜHRUNG ANTHROPOMETRISCHER MESSUNGEN**

(30) Priorität: 18.10.2006 CL 200602734
(71) Anmelder: Patrick H. Ballew, Inc. P.s., Yakima, WA 98902 (US); Murá, Yañez Miguel Angel, Santiago 341 1647 (CL)
(72) Erfinder: MURÁ, Yañez, Miguel Angel, Santiago 341 1647 (CL)
(74) Vertreter: Arth, Hans-Lothar
(86) Internationale Anmeldenummer: PCT/IB2006/004235
(87) Internationale Veröffentlichungsnummer: WO 2008/078148

(57) **Zusammenfassung**

Die vorliegende Erfindung betrifft ein systemisches Verfahren und eine Vorrichtung, die dazu in der Lage ist, die Teilmaße von Personen (Kindern und Erwachsenen) zu messen.

## Beschreibung

Die vorliegende Erfindung betrifft ein systemisches Verfahren und eine Vorrichtung, die dazu in der Lage ist, die Teilmaße von Personen (Kindern und Erwachsenen) zu messen.

Das Anwendungsgebiet der Erfindung gehört also zur Anthropometrie und insbesondere zu den anthropometrischen Untersuchungen, wobei die Messmannschaft diejenige ist, welche die Messung vornimmt, und wer gemessen wird, sind im Allgemeinen Bevölkerungssegmente oder -profile, wie beispielsweise Kinder eines bestimmten Altersbereichs oder Werktätige in einem bestimmten Unternehmen usw. Die repräsentativen Individuen werden mit bestimmten Zielen und Kriterien, die der Untersuchung eigen sind, gemessen. Die Untersuchungen, die sowohl durch ein einzelnes Individuum als auch durch eine große Zahl derselben gebildet werden, stellen ein Instrument dar, das dazu in der Lage und dafür bestimmt ist, eine Datenbasis zu gewinnen, die in quantitativen Begriffen Daten synthetisiert und darstellt, die einem Durchschnitt, einem Perzentil usw. entsprechen.

### STAND DER TECHNIK

In Anbetracht dessen, dass die Bevölkerung in Abmessungsbegriffen auf der Erde heterogen ist und sich in fortwährender Entwicklung befindet, und ihrer unmittelbaren Auswirkung, da sie es ist, die sich in den Gegenständen verbindet, dieselben beeinflusst und in denselben enthalten ist, ist das Messen und Kennen der Teilmaße unverzichtbar für diejenigen, welche die Abmessungsvariable in ihre Aufgaben einbeziehen. Dies ist unmittelbar der Fall der Produktgestalter, Architekten, Gestalter von Kleidungsstücken und Garderobe, Gestaltung von Prothesen, Bewertung oder Untersuchungen von Höchstleistungen auf dem Gebiet des Sports, Einrichtungen, die sich mit den Arbeitsbedingungen beschäftigen usw. Außerdem werden heute, da der Technologietransfer im Rahmen der Globalisierung stattfindet, diese Daten immer wichtiger und wertvoller, sowohl auf lokalem als auch auf internationalem Niveau.

Es gibt 31 oder 37 anthropometrische Maße (auf der Grundlage von: Précis de physiologie du travail, Notions d'ergonomie, J.Scherrer et collaborateurs, 2a edición, Editorial Masson, Paris, 1967, 1981 und Victoria Ratinoff Ferrera, Professorin für Ergonomie und Leiterin des Labors für Ergonomie der Universidad Tecnológica Metropolitana, ordentliches Mitglied des Nationalen Ergonomieausschusses), auf der Grundlage von anthropometrischen Punkten mit Bezügen auf die Skelettstruktur (äußere und innere) und anthropometrischen Betrachtungspositionen für die Individuen, die gemessen werden (stehend, sitzend und mit ausgebreiteten Armen).

Im Allgemeinen ist es so dass das Bevölkerungssegment oder -profil an dem Ort gemessen werden sollte, wo es seine Aufgaben erfüllt, zum Beispiel die Schüler in der Schule, der Werktätige im Unternehmen usw., weshalb sowohl die Messmannschaft als auch die Ausrüstung, über die sie verfügt, transportiert werden müssen.

Unter den digitalen Verfahren gibt es mehrere Geräte, die dazu in der Lage sind, den Teil und seine Strukturen mit einem hohen Niveau an Genauigkeit zu erfassen. Im Fall des Scanners sind sie nicht transportabel, und außerdem besitzt der Umfang an Informationen für diese Art von Untersuchungen, obwohl er nicht notwendig ist, einen Maßstab und eine Nacharbeit, die nicht angemessen sind. Die fotometrischen Kameras, obwohl sie auf Grund ihrer Augenblicklichkeit die beste Lösung scheinen und heutzutage technologisch genauer sind, treten den gemessenen Benutzer zu nahe insofern, als sie seine körperliche Privatsphäre einer Aufzeichnung und ihrer späteren Verarbeitung unterwerfen.

Die Messungen nach dem manuellen Verfahren, wobei das Erkennen und Lokalisieren des anthropometrischen Punkts in taktiler (manueller) Form erfolgt, sind am zuverlässigsten und genauesten, erfordern aber mehr Arbeit, Zeit und eine Mannschaft von mehreren Personen.

Das beim manuellen Verfahren benutzte Instrumentarium wird hauptsächlich von Folgendem gebildet: Martin- und Netz-Anthropometer, ausziehbare Tastzirkel, verschiebbarer Zirkel und Biegungsmesser (Bandmaß).

Es zeigt Probleme der folgenden Art:
- funktionelle Ermüdung wegen Instabilität des Instruments,
- unzureichende Handhabung in Bezug auf die Stabilität des Instruments und die Zuverlässigkeit der Ablesung,
- zeitaufwändig, die Messung durchzuführen, da 3 Personen (Messender, Protokollierender oder Assistent und die gemessene Person) koordiniert werden müssen,
- wesentliche Aggressivität (scharfe Kanten, Temperatur usw.) in Anbetracht dessen, dass Personen gemessen werden, die im Allgemeinen fast ohne Wäsche oder Kleidung sind,
- Fehlerquellen, sowohl bei der Übermittlung der Daten auf mündlichem Wege als auch bei der manuellen Transkription.

Logistisch gibt es physisch-administrative Probleme, verbunden mit der Verfügung über den physischen Ort von Seiten der Einrichtung, von der die gemessenen Individuen (Proben) genommen werden, auf Grund der Ungewissheit über die zum Durchführen der Messungen erforderliche Zeit.

Die für das Erledigen der Maßaufzeichnung eingerichteten Räume sind im Allgemeinen nicht die am besten geeigneten, da sie Unregelmäßigkeiten im Niveau des Fußbodens, der Wände und des Baus im Allgemeinen und sicher der Beleuchtung zeigen, was Fehlerquellen vermehrt, die sich akkumulierend und fortschreitend zum Wesentlichen der Messung, der Datenbasis und der zu ihrer Verwirklichung benötigten Zeit summieren.

Daher besteht auf dem Gebiet ein Bedarf an einer Vorrichtung, die auf systematische Weise, bei einfacher Benutzung, effiziente anthropometrische Daten vor Ort gewinnt.

### AUFGABEN DER ERFINDUNG

Der Zweck dieses Vorhabens besteht darin, quantitative Daten aus den anthropometrischen Messungen vor Ort zu gewinnen, die den Anforderungen von Zuverlässigkeit Schnelligkeit und räumlicher Verfügbarkeit entsprechen, wobei systematisch Folgendes berücksichtigt wird: Transport, Entfaltung und Nivellement des Produkts, Ausrichtung und Haltungseinstellung (gemessenes Individuum), manuelle taktile Lokalisierung (messendes Individuum), Aufzeichnung und Digitalisierung, Speicherung, Verarbeitung, Nachverarbeitung und Verteilung der anthropometrischen Daten.

Dies wird es möglicherweise erlauben, Datenbasen zu potenzieren und zu schaffen, wobei die Teilmaßparameter der Benutzergruppe in einer zuverlässigen und genauen Form innerhalb eines Projektvorgangs (im Fall des Gestalters) bereitgestellt werden. Dies wiederum trägt dazu bei, dass der Gestalter und andere Disziplinen (Medizin, Sport, Kleidung) den Faktor des menschlichen Maßes qualitativ bewerten.

### KURZE BESCHREIBUNG DER ZEICHNUNGEN

Weitere Kennzeichen und Vorzüge der Erfindung werden offensichtlich aus der folgenden Beschreibung ihrer bevorzugten Ausführungsform, die nur als erläuterndes und nicht begrenzendes Beispiel gegeben wird, unter Bezugnahme auf die beigefügten Zeichnungen, in denen:
Figur 1 das Diagramm des Verfahrens, das die vorliegende Erfindung darstellt, zeigt;
Figur 2 eine Seitenansicht der Schutzvorrichtungen, die in einem Fahrzeug mit dem Fassungsvermögen für die Erfindung transportiert werden, illustriert;
Figur 3 in Seitenansicht die manuell umgesetzten Schutzvorrichtungen der Erfindung illustriert;
Figur 4 eine perspektivische Vorderansicht der Schutzvorrichtung der Erfindung, die den Haltungsausrichter enthält, zeigt;
Figur 5 eine perspektivische Rückansicht der Schutzvorrichtung der Erfindung, die den Haltungsausrichter enthält, zeigt;
Figur 6 eine perspektivische Vorderansicht des gefalteten Haltungsausrichters der Erfindung zeigt;
Figur 7.1 eine perspektivische Rückansicht des sich entfaltenden Haltungsausrichters der Erfindung zeigt;
Figur 7.2 eine perspektivische Vorderansicht des entfalteten Haltungsausrichters der Erfindung zeigt;
Figur 7.3 eine perspektivische Rückansicht des entfalteten Haltungsausrichters der Erfindung zeigt;
Figur 8 eine perspektivische Vorderansicht der kartesischen Schutzvorrichtung der Erfindung, in geschlossenem Zustand, die den kartesischen Rahmen enthält, zeigt;
Figur 9 eine perspektivische Rückansicht der kartesischen Schutzvorrichtung der Erfindung, in geschlossenem Zustand, die den kartesischen Rahmen enthält, zeigt;
Figur 10 eine perspektivische Vorderansicht der kartesischen Schutzvorrichtung der Erfindung, in offenem Zustand, worin der gefaltete kartesische Rahmen herausgenommen wird, zeigt;
Figur 11 eine perspektivische Vorderansicht des kartesischen Rahmens der Erfindung, in gefaltetem Zustand, zeigt;
Figur 12 eine perspektivische Rückansicht des kartesischen Rahmens der Erfindung, in gefaltetem Zustand, zeigt;
Figur 13.1 eine perspektivische Vorderansicht des sich entfaltenden kartesischen Rahmens der Erfindung zeigt;
Figur 13.2 eine perspektivische Vorderansicht des kartesischen Rahmens der Erfindung, in entfaltetem Zustand, zeigt;
Figur 14 eine perspektivische Vorderansicht des Haltungsausrichters und des kartesischen Rahmens der Erfindung, in der Kopplungsphase, zeigt;
Figur 14.1 in perspektivischer Vorderansicht ein Detail der oberen Stütze der Erfindung zeigt;
Figur 14.2 in perspektivischer Vorderansicht ein Detail der Aufnahme der Erfindung zeigt;
Figur 15 eine perspektivische Vorderansicht des Haltungsausrichters, des kartesischen Rahmens und eines Notebooks mit bestimmten Kennzeichen, in der Phase der Datenübertragung und des Nivellements, für die mögliche Benutzung der Erfindung zeigt;
Figur 15.1 eine perspektivische Vorderansicht des Haltungsausrichters, des kartesischen Rahmens und eines Notebooks mit bestimmten Kennzeichen der Erfindung, in der Phase der Übertragung der Messung, verwendet durch beide Benutzer, zeigt;
Figur 16 ein erläuterndes Detail der perspektivischen Vorderansicht, das dem Muster-Messverfahren der Erfindung entspricht, das am gemessenen Benutzer (8) durch einen Anthropometristen, den messenden Benutzer (9), ausgeführt wird, zeigt;
Figur 17 eine perspektivische Frontansicht zeigt, wobei eine Mustermessung der Erfindung ausgeführt wird, wobei der kartesische Rahmen in die Position Vorderansicht gedreht ist;
Figur 18 eine perspektivische Frontansicht zeigt, wobei eine Mustermessung der Erfindung ausgeführt wird, wobei der kartesische Rahmen in die Position rechte Seitenansicht gedreht ist;
Figur 19 eine perspektivische Frontansicht zeigt, wobei eine Mustermessung der Erfindung ausgeführt wird, wobei der kartesische Rahmen in die Position Rückansicht gedreht ist;
Figur 20 eine perspektivische Frontansicht zeigt, wobei eine Mustermessung der Erfindung ausgeführt wird, wobei der kartesische Rahmen in die Position linke Seitenansicht gedreht ist;
Figur 21 eine Ansicht im Aufriss von oben zeigt, wobei eine schräge horizontale Messung der Erfindung ausgeführt wird;
Figur 22 eine senkrechte Vorderansicht der Erfindung, wobei der kartesische Rahmen in Sagittalposition gedreht ist, wobei eine schräge vertikale Messung der Erfindung ausgeführt wird;
Figur 23 eine senkrechte Vorderansicht zeigt, wobei der kartesische Rahmen in Sagittalposition gedreht ist, wobei eine ausgerichtete horizontale Messung der Erfindung ausgeführt wird;
Figur 24 eine perspektivische Seitenansicht zeigt, wobei eine Mustermessung der Erfindung ausgeführt wird, angeordnet zum Aufnehmen der stehenden Haltung;
Figur 25 eine perspektivische Seitenansicht zeigt, wobei eine Mustermessung der Erfindung ausgeführt wird, angeordnet zum Aufnehmen der sitzenden Haltung;
Figur 26 eine perspektivische Seitenansicht zeigt, wobei eine Mustermessung der Erfindung ausgeführt wird, angeordnet zum Aufnehmen der Haltung mit ausgebreiteten Armen;
Figur 27 eine auseinandergezogene perspektivische Vorderansicht der Hauptbestandteile der Erfindung zeigt;
Figur 28 eine obere perspektivische Vorderansicht der oberen Stützbaugruppe (3.1) der Erfindung zeigt;
Figur 29 eine untere perspektivische Vorderansicht der oberen Stützbaugruppe (3.1) der Erfindung zeigt;
Figur 30 ein Detail des Schnitts A der oberen Ansicht der oberen Stützbaugruppe (3.1) der Erfindung zeigt;
Figur 32 ein Detail von Figur 31 zeigt, eine aufgebrochene Ansicht in Vorderansicht der oberen Stützbaugruppe (3.1) der Erfindung;
Figur 33 eine perspektivische Vorderansicht der Säulenbaugruppe (3.3) der Erfindung zeigt;
Figur 34 eine perspektivische Rückansicht der Säulenbaugruppe (3.3) der Erfindung zeigt;
Figur 35 eine perspektivische Vorderansicht der Armstützenbaugruppe (3.4) der Erfindung zeigt;
Figur 36 eine perspektivische Unteransicht der Armstützenbaugruppe (3.4) der Erfindung zeigt;
Figur 37 eine perspektivische Vorderansicht der Sitzbaugruppe (3.5) der Erfindung zeigt;
Figur 38 eine perspektivische Unteransicht der Sitzbaugruppe (3.5) der Erfindung zeigt;
Figur 39 eine perspektivische Vorderansicht der unteren Stützbaugruppe (3.2) der Erfindung zeigt;
Figur 40 eine perspektivische Unteransicht der unteren Stützbaugruppe (3.2) der Erfindung zeigt;
Figur 41 eine perspektivische Vorderansicht der Rahmenbaugruppe (4.1) der Erfindung zeigt;
Figur 42 eine perspektivische Rückansicht der Rahmenbaugruppe (4.1) der Erfindung zeigt;
Figur 43 eine perspektivische Vorderansicht der horizontalen Achsbaugruppe (4.2) der Erfindung zeigt;
Figur 44 eine perspektivische Rückansicht der horizontalen Achsbaugruppe (4.2) der Erfindung zeigt;
Figur 45 eine perspektivische Vorderansicht der kartesischen Hauptmanipulatorbaugruppe (4.3) der Erfindung zeigt;
Figur 46 eine perspektivische Vorderansicht der kartesischen Nebenmanipulatorbaugruppe (4.4) der Erfindung zeigt.

### BESCHREIBUNG DER BEVORZUGTEN AUSFÜHRUNGSFORM

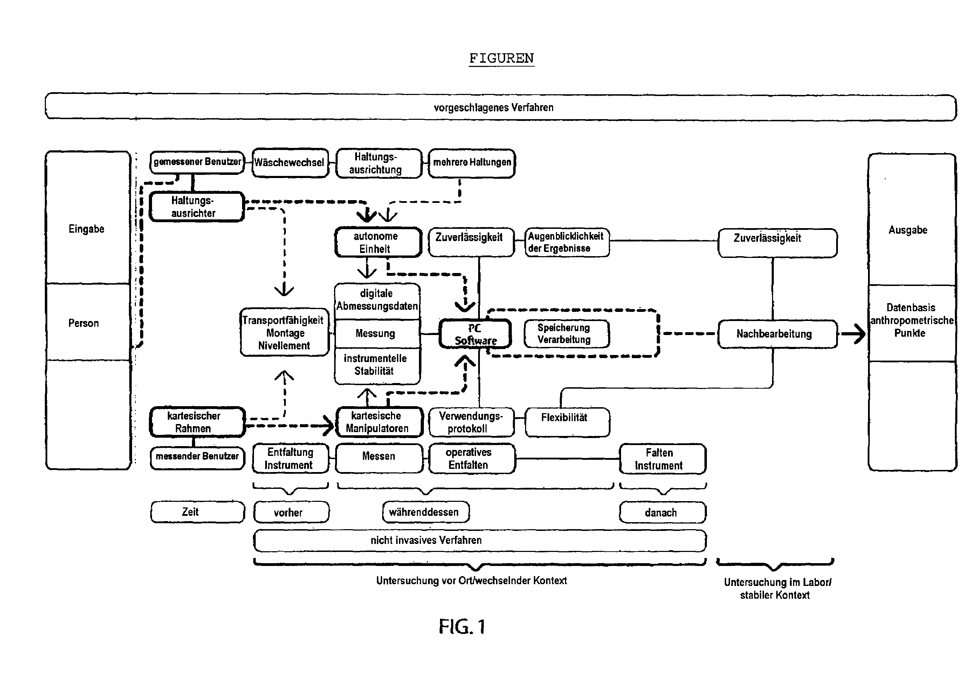

Um die ausführliche Beschreibung der bevorzugten Ausführungsform der Vorrichtung der Erfindung durchzuführen, wird fortlaufend Bezug auf die Figuren der Zeichnungen genommen, wobei Figur 1 das Übersichtsdiagramm des vorgeschlagenen Verfahrens ist, in dessen System wir als Eingabe das zu messende Individuum und als Ausgabe die Datenbasis haben, die von den beachtenswerten anthropometrischen Punkten abgeleitet wird, die von diesem abgenommen werden. Es wird von zwei Untersystemen dargestellt, das erste, das Haltungsausrichter genannt wird, da es im Allgemeinen ein Haltungsprotokoll ausrichten und aufnehmen soll, und das zweite Untersystem, das kartesischer Rahmen genannt wird, welches die Betriebsfunktionen des Individuums, das die Messung vornimmt, aufnimmt. Diese komplementären Einheiten werden in einem Fahrzeug mittleren Fassungsvermögens transportiert, enthalten in Schutzvorrichtungen, um dann, bereits vor Ort, in unabhängiger Form entfaltet zu werden, um sich danach zu koppeln. Während der Messung werden die Informationen an eine Software gesendet, die in digitaler Form sowohl das Aufzeichnen, Speichern, Verarbeiten, Übergeben der Ergebnisse als auch das Nachbearbeiten ermöglicht.

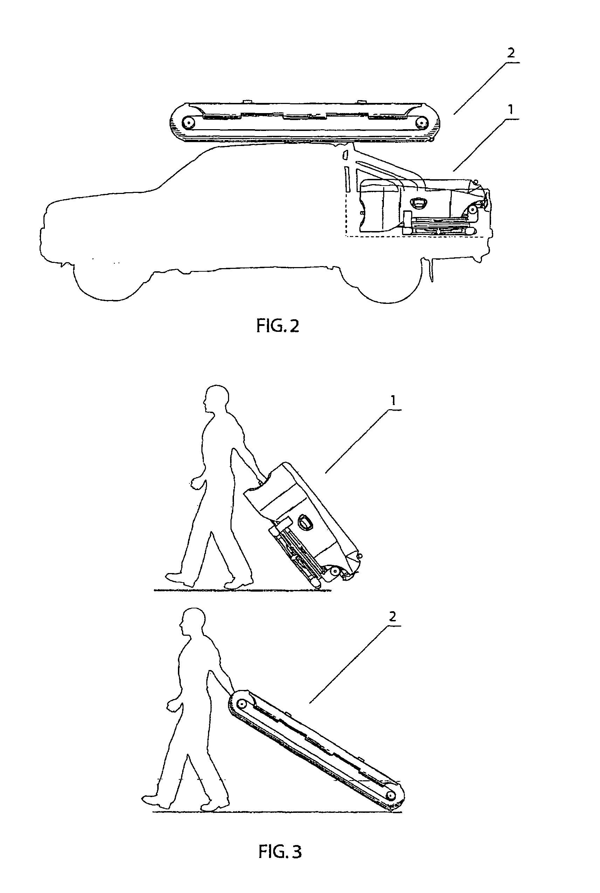

Figur 2. In dieser Figur ist zu sehen, wie der kartesische Rahmen in seiner entsprechenden Schutzvorrichtung (2) auf dem Fahrzeug mittlerer Traglast angeordnet ist und seinerseits der Haltungsausrichter in seiner entsprechenden Schutzvorrichtung (1).

Figur 3. In dieser Figur ist zu sehen wie Haltungsausrichter in seiner entsprechenden Schutzvorrichtung (1) und der kartesische Rahmen (2), jeweils in ihren Schutzvorrichtungen umgesetzt werden.

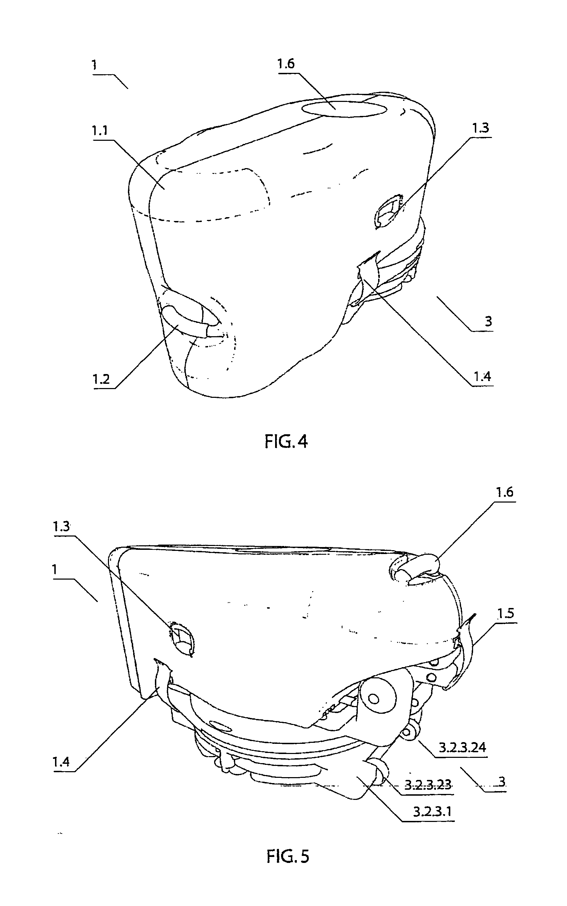

Figur 4. In dieser Figur ist der Haltungsausrichter in seiner entsprechenden Schutzvorrichtung (1) zu sehen, wobei der Hauptteil oder das Gehäuse (1.1) dazu in der Lage ist, den Abschnitt abzudecken, der am empfindlichsten für Schläge von Umsetzen und Transport ist, von dessen Vorderteil sich ein konkaver Raum erstreckt, wo in horizontaler Form ein Handgriffelement (1.2) für dessen Umsetzung angeschlossen ist. Auf beiden seitlichen Seiten erstreckt sich wiederum eine Rundhöhlung, in der ein greifbares schwenkbares Element (1.3) verbunden ist. Außerdem sind in die Schutzvorrichtung drei Angelverankerungen (1.4) eingeschlossen, die dazu in der Lage sind, die Verbindung zwischen diesem und dem Haltungsausrichter zu sichern, wobei eine derselben in der folgenden Figur ausführlich zu erkennen ist. Schließlich erstreckt sich im oberen Bereich eine kreisförmige Aussparung (1.6), für den Einschluss eines Bildes.

Figur 5. In dieser Figur sind, zugehörig zum Gehäuse, die dritte Angelverankerung (1.5), die hintere Rundhöhlung, in der horizontal ein Handgriffelement (1.6) untergebracht ist, und schließlich, zugehörig zum Haltungsausrichter, in einem ausgesparten Abschnitt der Basis (3.2.3.1) zwei Rollen (3.2.3.23) und (3.2.3.24), die sich zum Inneren hin erstrecken und einzeln verbunden sind, zu sehen.

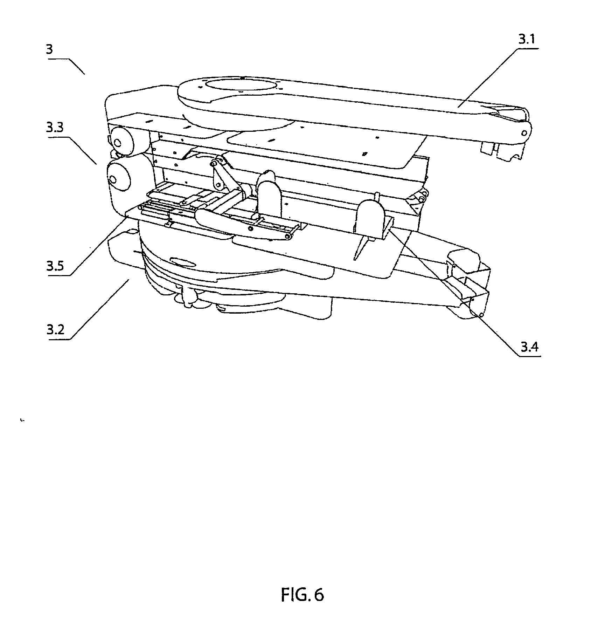

Figur 6. In dieser Figur ist der Haltungsausrichter ohne seine Schutzvorrichtung vollständig gefaltet zu sehen, wobei die obere Stütze (3.1), die untere Stütze (3.2), die Säule (3.3), die Armstütze (3.4) und der Sitz (3.5) gefaltet, eingezogen und zusammengelegt sind.

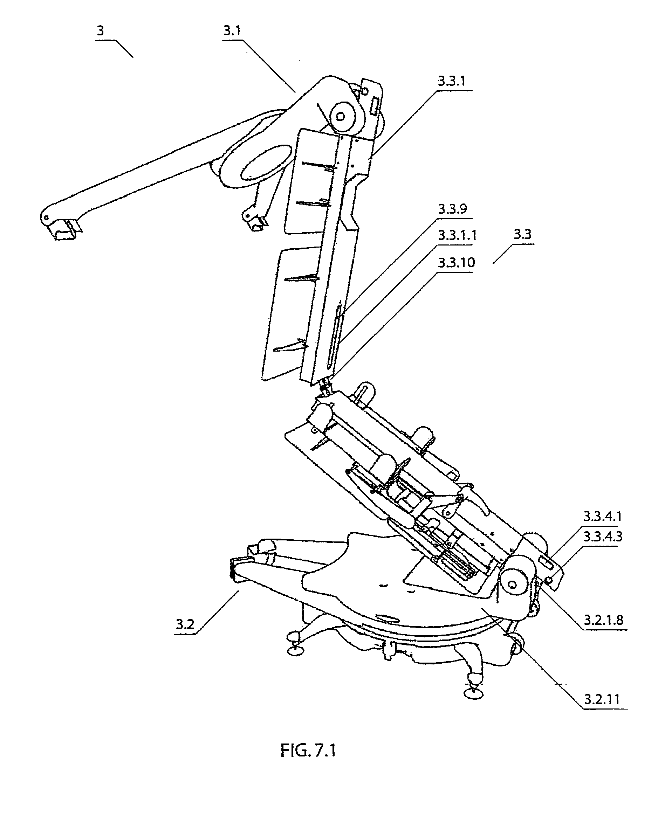

Figur 7.1. In dieser Figur sind zu sehen: ein kleiner Teil (3.3.9), dazu in der Lage, sich in dem länglichen Abschnitt (3.3.1.1) zu verschieben, der von dem großen ausgehöhlten Teil ausgespart ist (3.3.1), ein Scharnier (3.3.10), ein kleiner Teil, der die zwei großen ausgehöhlten Teil (3.3.1) und (3.3.2) verbindet, wobei diese großen Teil dazu in der Lage sind, in einer Horizontalachse und senkrecht zu ihren Leitlinien der Entfaltungsbewegung zu schwenken. An den äußersten Enden dieser großen ausgehöhlten Teil befinden sich ein Stellglied (3.3.4.1), ein Effektor (3.3.4.3), der im Augenblick seiner Einstellung in ein Gegenstück (3.2.1.8) eingeführt wird, wobei das letztere in einer Plattform (3.2.11) angeordnet ist.

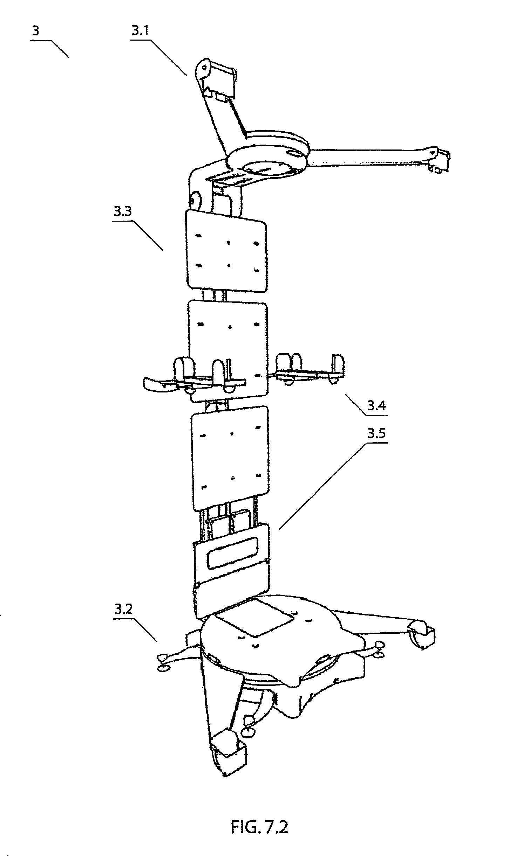

Figur 7.2. In dieser Figur ist der Haltungsausrichter in perspektivischer Vorderansicht vollständig entfaltet zu sehen, wobei die obere Stütze (3.1) und die untere Stütze (3.2) durch die Säule (3.3) vereint und gelenkig gelagert werden, an der die Armstütze (3.4) und der Sitz (3.5) angeordnet sind.

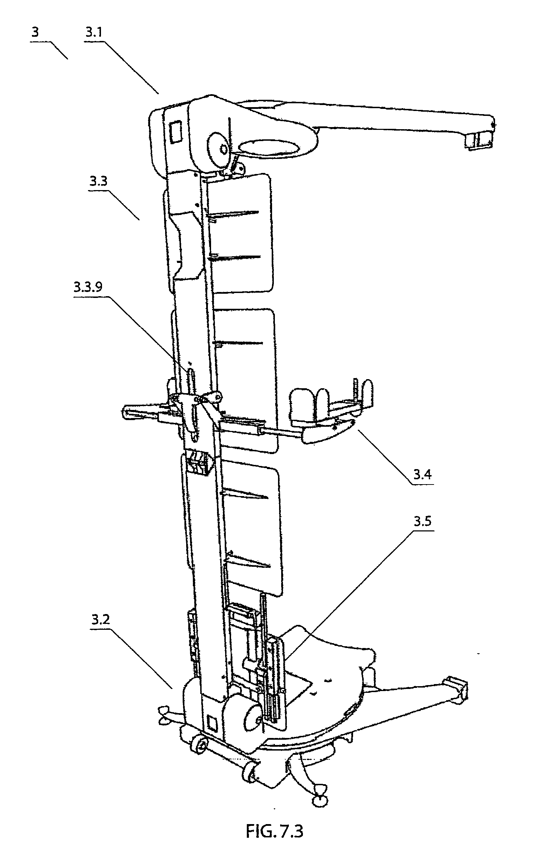

Figur 7.3. In dieser Figur ist der Haltungsausrichter in perspektivischer Rückansicht vollständig entfaltet zu sehen, wobei wie in Figur 7.2 die obere Stütze (3.1) und die untere Stütze (3.2) durch die Säule (3.3) vereint und gelenkig gelagert werden, an der die Armstütze (3.4) und der Sitz (3.5) angeordnet sind.

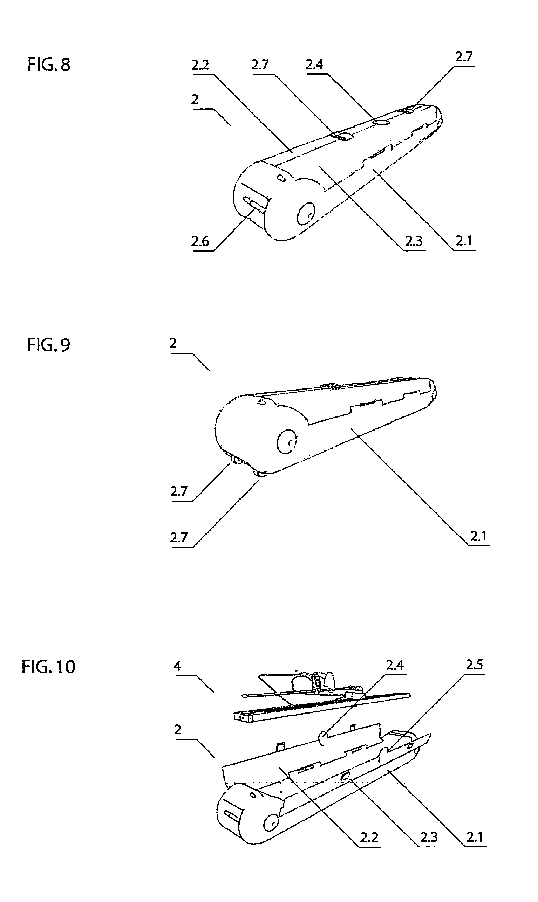

Figur 8. In dieser Figur ist die Schutzvorrichtung des kartesischen Rahmens in perspektivischer Vorderansicht zu sehen, wobei es den großen ausgehöhlten Raum (2.1) gibt, in dem sich am Rand seines ausgehöhlten Abschnitts schwenkbare Teil an seiner Basis (2.2 und 2.3) verbinden. Zwei Angelverankerungen (2.7) ermöglichen es, die zwei bereits erwähnten schwenkbaren Teil zeitweilig zu verbinden. Schließlich gibt es eine Rundhöhlung, in der in horizontaler Form eine Rolle (2.6) angeschlossen ist.

Figur 9. In dieser Figur ist die Schutzvorrichtung des kartesischen Rahmens in perspektivischer Rückansicht zu sehen, wobei es am unteren Teil eine Rundhöhlung gibt, wobei an der Seitenfläche jeweils eine Rolle (2.7) angeschlossen ist.

Figur 10. In dieser Figur ist die perspektivische Vorderansicht der kartesischen Schutzvorrichtung, in offenem Zustand, zu sehen, wobei der gefaltete kartesische Rahmen herausgenommen ist; wobei die kleinen Teil (2.2 und 2.3) zu diesem Zweck geschwenkt haben. Der halbkreisförmige Abschnitt (2.4), der über den zweiten (2.5) vorspringt, ist besser zu erkennen.

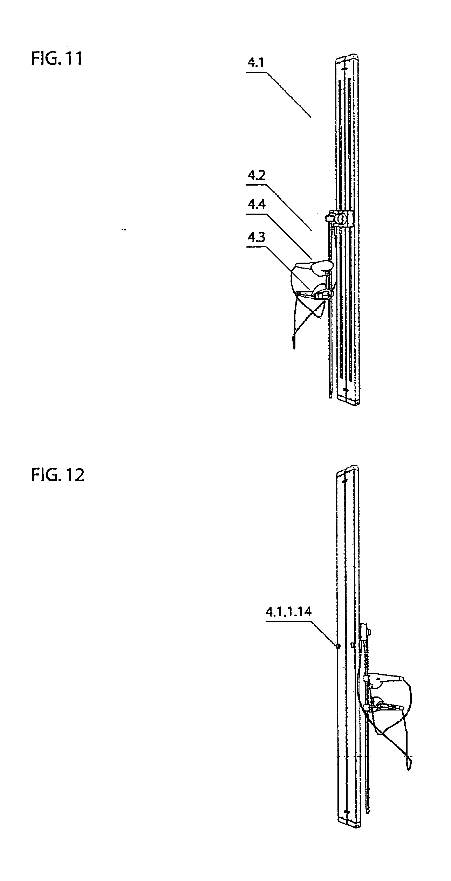

Figur 11. In dieser Figur ist in perspektivischer Vorderansicht der kartesische Rahmen, in gefaltetem Zustand, zu sehen, wobei die röhrenförmige horizontale Achse (4.2) den Hauptmanipulator (4.3) und den Nebenmanipulator (4.4) anordnet.

Figur 12. In dieser Figur ist eine perspektivische Rückansicht des kartesischen Rahmens, in gefaltetem Zustand, zu sehen, wobei die Stellglieder (4.1.1.14) aus dem Inneren erscheinen, in symmetrischer Form angeordnet in der Mitte einer jeden der erwähnten Flächen.

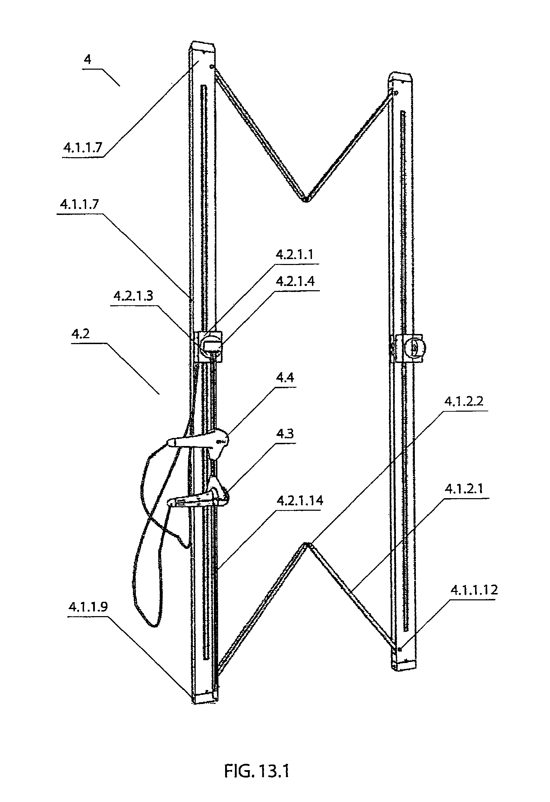

Figur 13.1. In dieser Figur ist eine perspektivische Vorderansicht des sich entfaltenden kartesischen Rahmens (4), zu sehen, wobei der gefaltete laminare Teil (4.1.2.1), der an seiner unteren und äußeren Basis an einer Achse schwenkt, die mit einer Bohrung (4.1.1.12) verbunden ist, die in einem vertikalen großen ausgehöhlten Teil (4.1.1.2) angeordnet ist, was ihr mögliches Zurückhalten ermöglicht, und an seinem oberen und inneren Ende gelenkig mit einem anderen kleinen laminaren Teil (4.1.2.2) verbunden ist.

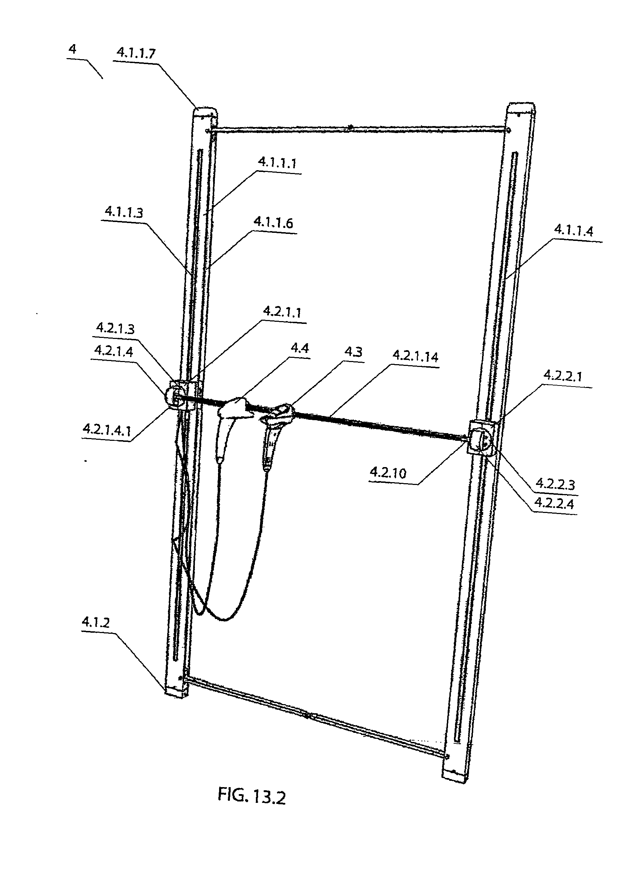

Figur 13.2. In dieser Figur ist eine perspektivische Vorderansicht des kartesischen Rahmens (4), in entfaltetem Zustand, zu sehen, hauptsächlich bestehend aus dem Rahmen (4.1) und der horizontalen Achse (4.2), wobei die vertikale Achse (4.1.1.1), ein ausgehöhlter Teil, einen Ausschnitt (4.1.1.3) längs ihres vorderen Teils und einen weiteren auf ihrer seitlichen Innenfläche (4.1.1.6) aufweist. Die horizontale Achse (4.2) ist dazu in der Lage, sich über den Rahmen (4.1) in ihrer Hauptachsenrichtung zu verschieben. Das vielflächige ausgehöhlte Volumen (4.2.11) integriert das angrenzende zylindrische Volumen (4.2.1.3), was dessen axiale Drehung ermöglicht, dieses wiederum integriert das anschließende halbzylindrische ausgehöhlte Volumen (4.2.1.4). Das letztere nimmt auf seiner inneren Seitenfläche einen laminaren Teil (4.2.1.14) auf, mittels eines zylindrischen Teils, das sie verbindet (4.2.1.4.1). Am entgegengesetzten Ende erstreckt sich das vielflächige ausgehöhlte Volumen (4.2.2.1), das ein angrenzendes zylindrisches Volumen integriert (4.2.2.3) über das sich in einer horizontalen Richtung das anschließende halbzylindrische Volumen (4.2.2.4) verschiebt, mit dem Ziel, die Schwenkposition der Baugruppe für deren abschließende Entfaltungsetappe zu sichern. Der laminare Teil (4.2.1.14) ermöglicht, dass sich sowohl der Hauptmanipulator (4.3) als auch der Nebenmanipulator (4.4) in ihrer Hauptachsenrichtung verschieben.

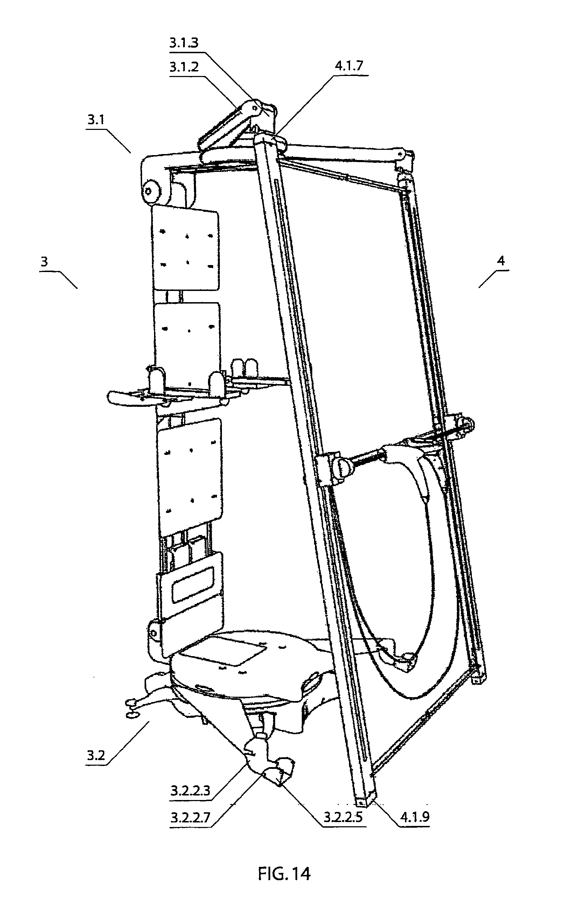

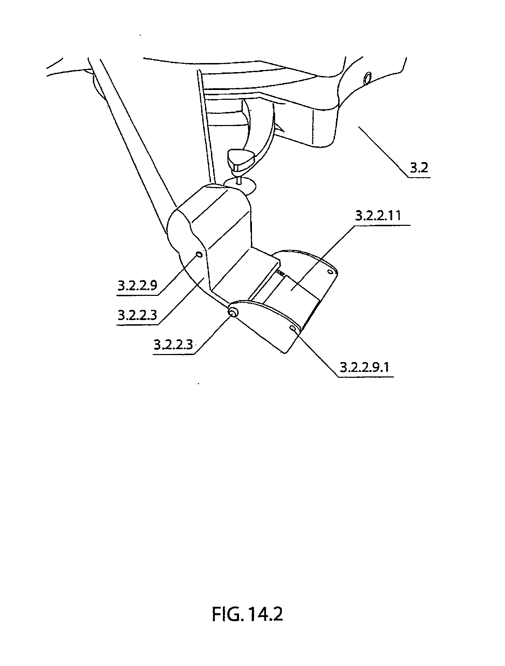

Figur 14. In dieser Figur ist die perspektivische Vorderansicht des Haltungsausrichters (3) und des kartesischen Rahmens (4) zu sehen, in der Kopplungsphase, einem durch ein Individuum ausgeführten Vorgang, für den zwei aufeinanderfolgende Vorgänge ausgeführt werden. Zuerst muss der abgestumpfte Spitzteil (4.1.7), der zu (4) gehört, zum Inneren des laminaren Teils hin und in dessen Richtung ausgerichtet werden, einer oberen Aufnahme (3.1.3), die in (3.1.2) schwenkt. Zweitens muss am unteren Ende der ausgehöhlte Teil (4.1.9) zum Teil (3.2.2.3) hin und in dessen Richtung ausgerichtet werden, um danach mittels eines laminaren Teils (3.2.2.5), schwenkbar bei (3.2.2.7), befestigt zu werden, der in dem bereits erwähnten Teil (3.2.2.3) angeordnet ist, der ein selbsttätiges inneres Befestigungssystem aufweist.

Figur 14.2. In dieser Figur ist in perspektivischer Vorderansicht ein Detail der Aufnahme zu sehen, wobei sich eine Platte mit einer bestimmten Dicke (3.2.2.5.1) an der Innenseite von (3.2.2.5) erstreckt. Die Bohrung (3.2.2.9), die an der Seitenfläche von (3.2.2.5) und nahe dem Rand angeordnet ist, stimmt in ihrer Verschlussphase mit dem aus dem Inneren von (3.2.2.3) erscheinenden Teil (3.2.2.9.1) überein.

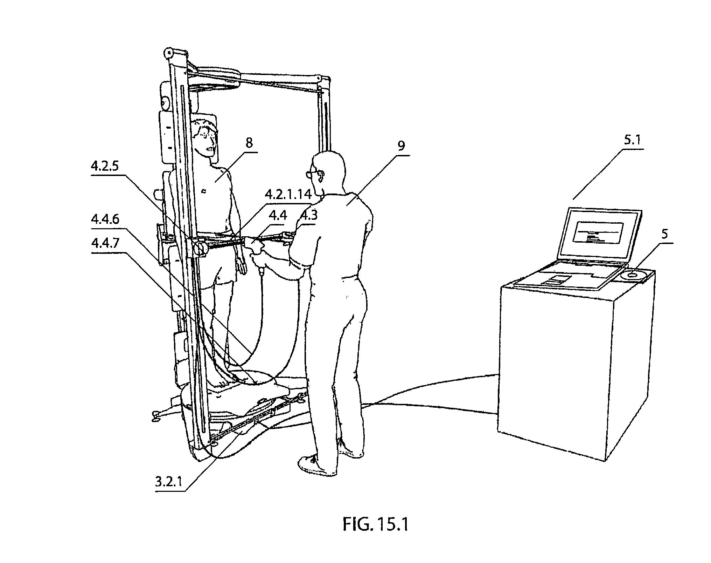

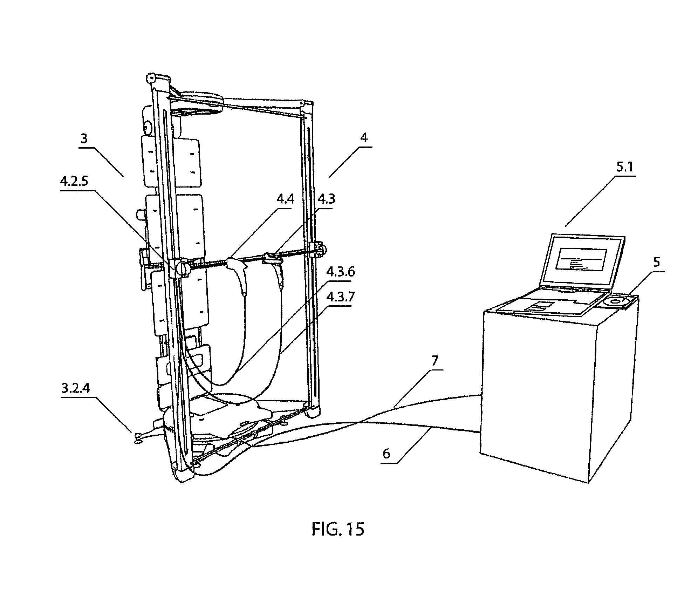

Figur 15. In dieser Figur ist eine perspektivische Vorderansicht des Haltungsausrichters (3), des kartesischen Rahmens (4), wobei die komplementären Einheiten gekoppelt sind, und eines Musternotebooks (5.1), in dem die Software (5) mit bestimmten Kennzeichen zu erkennen ist, zu sehen, in der Phase der Datenübertragung und des Nivellements. Dabei wird das letztere, nachdem es installiert und konfiguriert ist, sowohl mit Energie speisen als auch die anthropometrischen Daten aufzeichnen, speichern, verarbeiten und nachbearbeiten, die von den Einheiten (4 und 3) übertragen werden. In erster Instanz übergeben die Manipulatoren die Informationen an den vielflächigen ausgehöhlten Teil (4.2.5), der Schaltungen enthält, mittels geeigneter Kabel (4.3.7) für den Hauptmanipulator (4.3) und (4.4.6) für den Nebenmanipulator (4.4). Schließlich ist von dem vielflächigen ausgehöhlten Teil (4.2.5) ein geeignetes Schnittstellenkabel (6) angeschlossen und von der Basis (3.2.1) auf gleiche Weise (7).

Figur 15.1. In dieser Figur ist eine perspektivische Vorderansicht des Haltungsausrichters (3), des kartesischen Rahmens (4), wobei die komplementären Einheiten gekoppelt sind, und eines Musternotebooks (5.1), in dem die Software (5) mit bestimmten Kennzeichen zu erkennen ist, zu sehen, in der Phase der Datenübertragung und des Nivellements, die außerdem die Benutzer beim Benutzen des Systems zeigt, wobei sich der zu messende Benutzer (8) in einer Musterposition der anthropometrischen Betrachtung (stehend) befindet, auf genommen durch den bereits erwähnten Haltungsausrichter (3), und der Benutzer, der die Messung durchführt (9), den Nebenmanipulator (4.4) und den Hauptmanipulator (4.3) handhabt, die in dem kartesischen Rahmen (4) untergebracht sind.

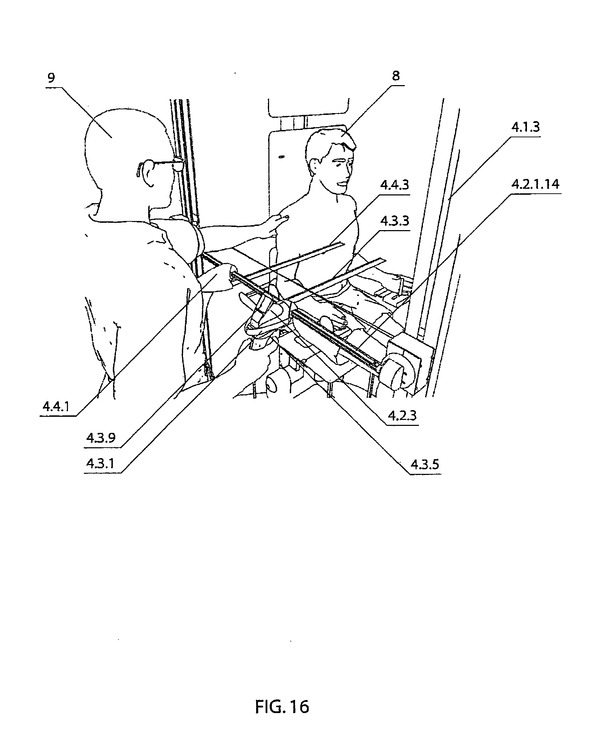

Figur 16. In dieser Figur ist das erläuternde Detail der perspektivischen Vorderansicht zu sehen, das dem Verfahren der Mustermessung entspricht, das am gemessenen Benutzer (8) durch einen Anthropometristen, den messenden Benutzer (9), ausgeführt wird, wobei an der horizontalen Achse (4.2) der Nebenmanipulator (4.4) in horizontaler Richtung verschoben werden kann, aus dessen großem ausgehöhlten Teil, dem Gehäuse (4.4.1), aus dessen Vorderteil ein sichtbares Lichtbündel (4.4.3) austritt, das sich senkrecht zur horizontalen Achse (4.2) erstreckt. Der Hauptmanipulator (4.3), umfasst, symmetrisch zum vorherigen anterior, außerdem, untergebracht im oberen Teil des großen ausgehöhlten Teils (4.3.1), eine schwenkbare digitale Visualisierungsvorrichtung für metrologische Informationen (4.3.9), die unter Umständen mittels eines taktilen Drückens eines zuvor mit der Hand ergriffenen Steuergeräts (4.3.5) aufgezeichnet werden.

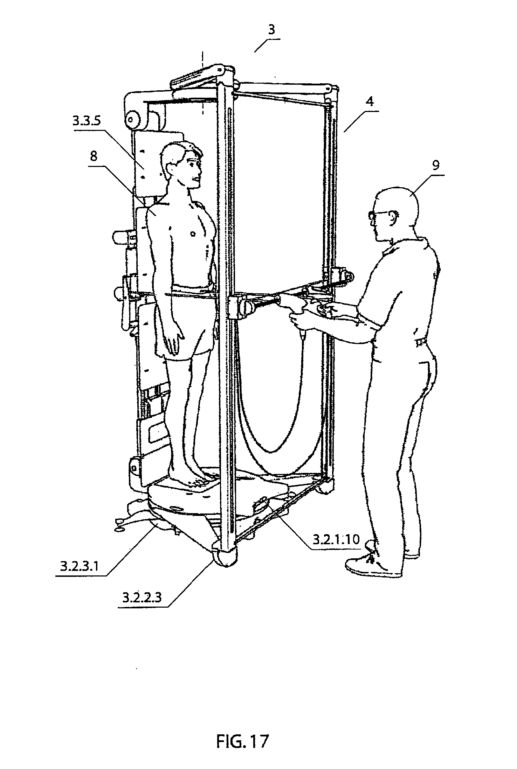

Figur 17. In dieser Figur ist eine perspektivische Frontansicht zu sehen, in der eine Mustermessung vorgenommen wird, wobei der kartesische Rahmen (4) in die Position Vorderansicht gedreht ist, parallel zur Ebene von (3.3.5), aufgenommen durch Aufnahmen (3.2.2.3), entfernt von und koaxial zu (3.2), in der Position gesichert durch einen Antrieb (3.2.1.10).

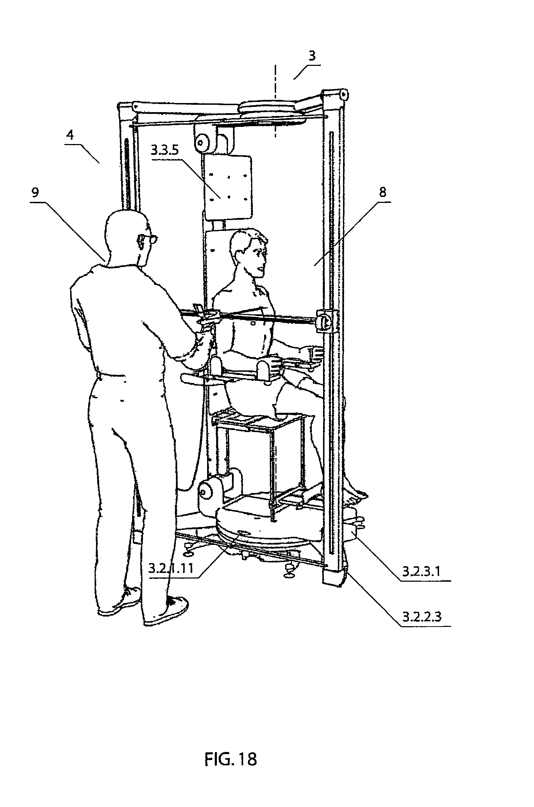

Figur 18. In dieser Figur ist eine perspektivische Frontansicht zu sehen, wobei der kartesische Rahmen (4) um 90° nach rechts in Bezug auf die Ebene von (3.3.5) gedreht ist, aufgenommen durch Aufnahmen (3.2.2.3), entfernt von und koaxial zu (3.2.3.1), in der Position gesichert durch einen Antrieb (3.2.1.11).

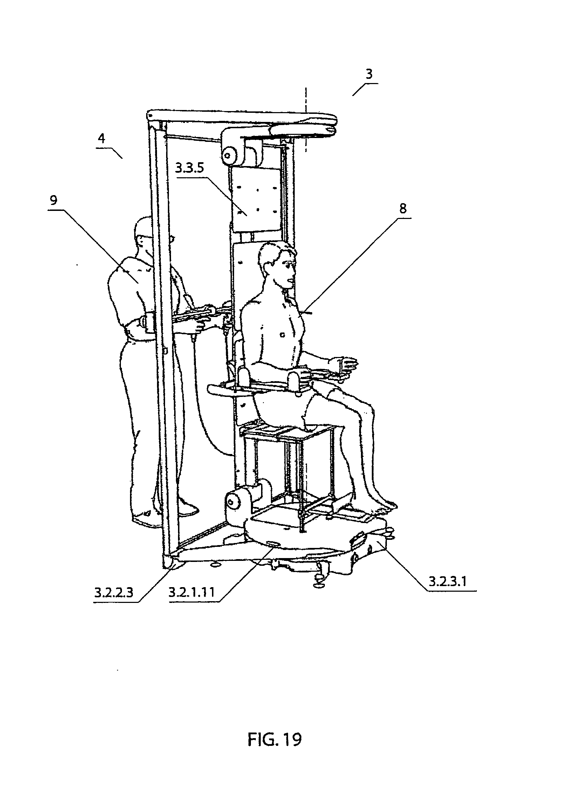

Figur 19. In dieser Figur ist eine perspektivische Frontansicht zu sehen, wobei der kartesische Rahmen (4) um 180° nach rechts in Bezug auf die Ebene von (3.3.5) gedreht ist, aufgenommen durch Aufnahmen (3.2.2.3), entfernt von und koaxial zu (3.2.3.1), in der Position gesichert durch einen Antrieb (3.2.1.11).

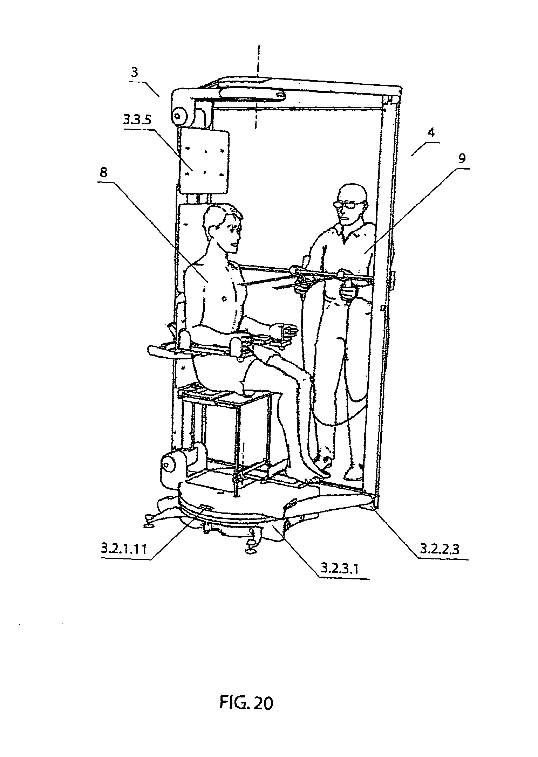

Figur 20. In dieser Figur ist eine perspektivische Frontansicht zu sehen, wobei der kartesische Rahmen (4) um 270° nach rechts in Bezug auf die Ebene von (3.3.5) gedreht ist, aufgenommen durch Aufnahmen (3.2.2.3), entfernt von und koaxial zu (3.2.3.1), in der Position gesichert durch einen Antrieb (3.2.1.11).

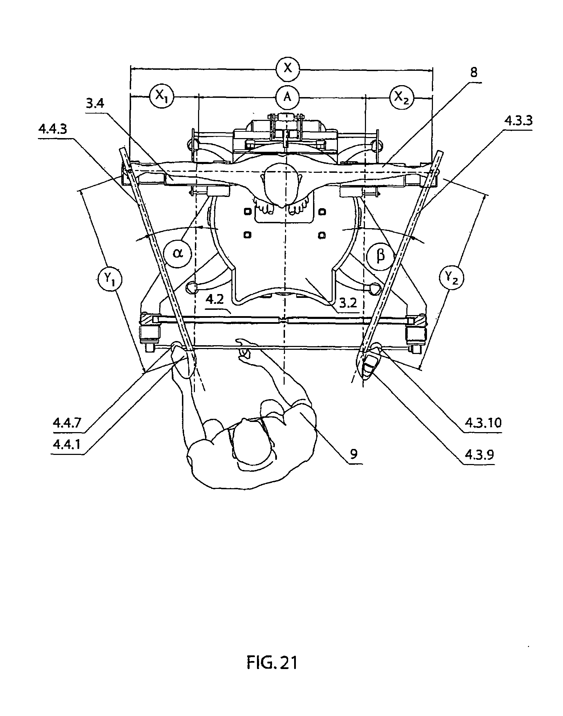

Figur 21. In dieser Figur ist eine Ansicht im Aufriss von oben zu sehen, in der eine schräge horizontale Messung vorgenommen wird, wobei der Nebenmanipulator (4.4.1) dazu in der Lage ist, an einem Drehteil (4.4.7) zu schwenken, wobei das sichtbare Lichtbündel (4.4.3) einen Winkel Alpha in Bezug auf die sagittale Symmetrieachse der Einheit umfasst. Symmetrisch dazu schwenkt der Hauptmanipulator (4.3.9) an (4.3.10), wobei er ebenfalls ein sichtbares Lichtbündel (4.3.3) auswirft, das in Bezug auf die sagittale Symmetrieachse der Einheit einen Winkel Beta bildet.

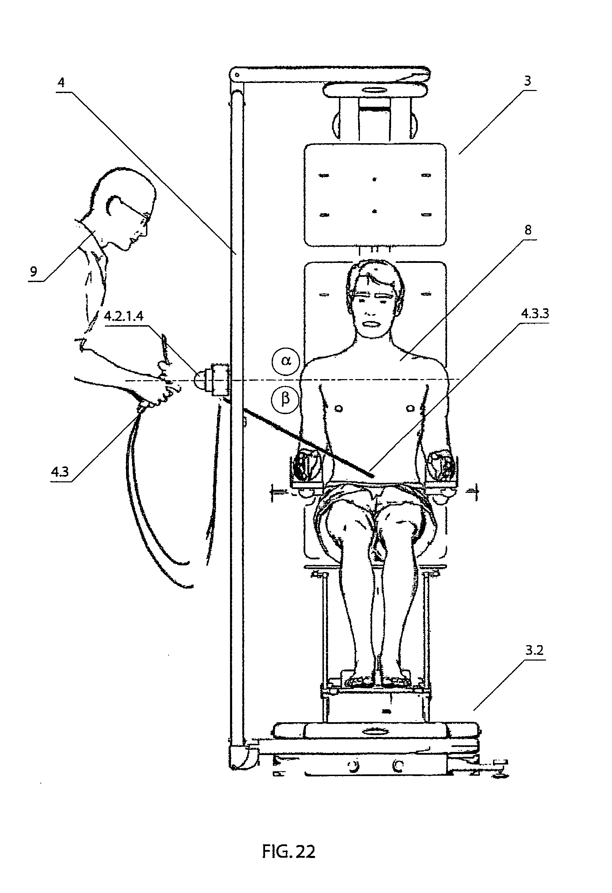

Figur 22. In dieser Figur ist eine senkrechte Vorderansicht zu sehen, wobei der kartesische Rahmen in eine sagittale Position gedreht ist, in der eine vertikale schräge Messung vorgenommen wird, wobei sich die kartesischen Manipulatoren um (4.2.1.4) drehen und für das sichtbare Lichtbündel (4.3.3) in Bezug auf eine horizontale Achse einen positiven Winkel Alpha und einen negativen Winkel Beta bilden.

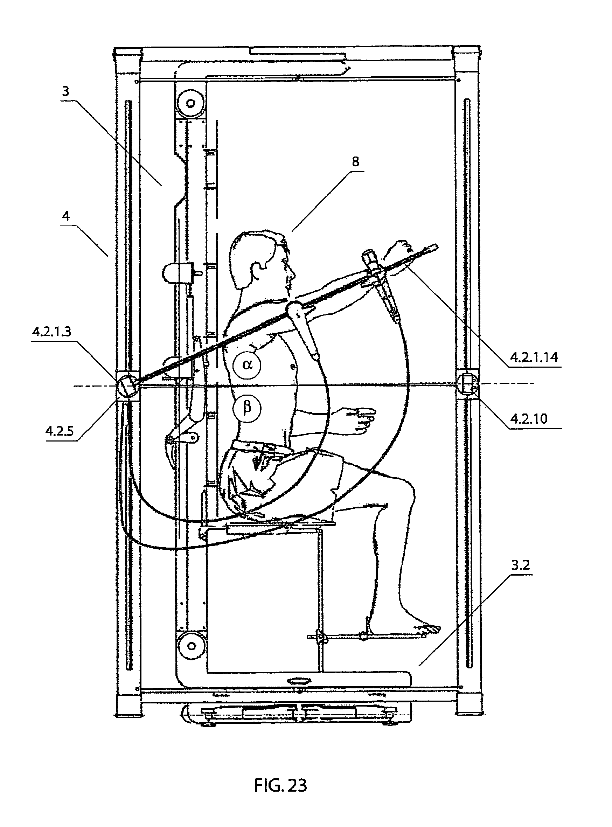

Figur 23. In dieser Figur ist eine senkrechte Seitenansicht zu sehen, wobei der kartesische Rahmen in eine sagittale Position gedreht ist, in der eine horizontale ausgerichtete Messung vorgenommen wird, wobei der laminare Teil (4.2.1.14), an dem die kartesischen Manipulatoren angeordnet sind, dazu in der Lage ist, koaxial zu dem zylindrischen ausgehöhlten Teil (4.2.1.3) zu schwenken und den positiven Winkel Alpha und den negativen Winkel Beta zu bilden.

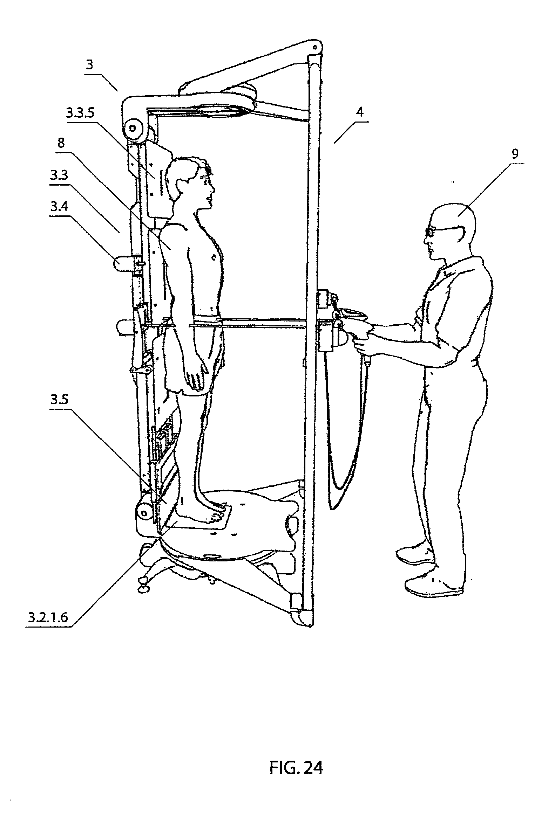

Figur 24. In dieser Figur ist eine perspektivische Seitenansicht zu sehen, in der eine Mustermessung vorgenommen wird, angeordnet zum Aufnehmen der stehenden Haltung, wobei der gemessene Benutzer (8) in der protokollarischen stehenden Haltung durch die Ebene der Säule (3.3) gestützt und durch den eingezogenen und gefalteten Sitz (3.5) aufgenommen wird. Außerdem ist ein rechteckiger Teil (3.2.1.6) zu erkennen, der bei (3.2) an die obere Fläche angesetzt ist.

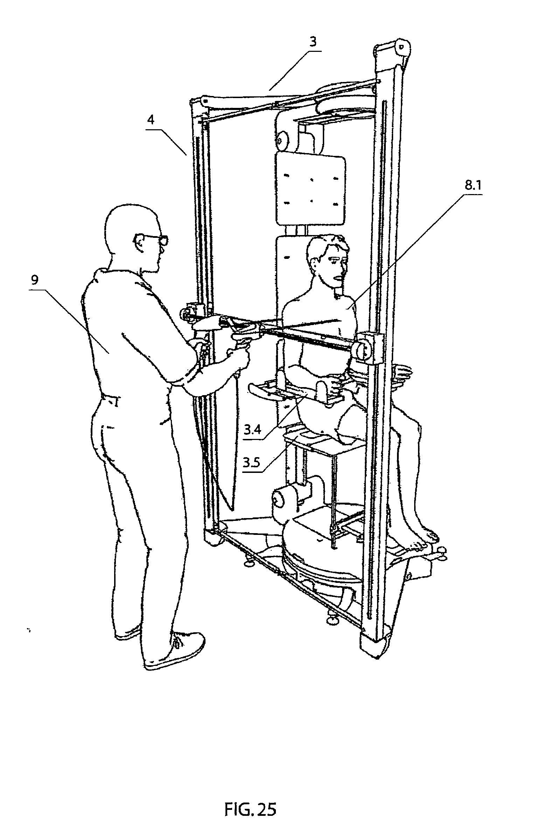

Figur 25. In dieser Figur ist eine perspektivische Seitenansicht zu sehen, in der eine Mustermessung vorgenommen wird, angeordnet zum Aufnehmen der sitzenden Haltung, wobei der gemessene Benutzer in sitzender Position (8.1) durch die Armstütze (3.4) und den Sitz (3.5) aufgenommen wird, die sich verlängern, drehen, umklappen und einstellen lassen.

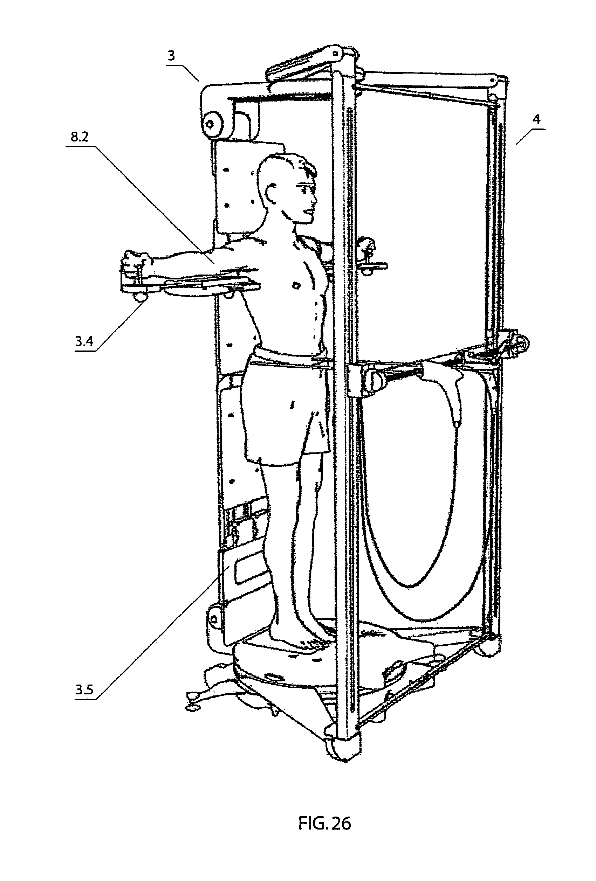

Figur 26. In dieser Figur ist eine perspektivische Seitenansicht zu sehen, in der eine Mustermessung vorgenommen wird, angeordnet zum Aufnehmen der Haltung mit ausgebreiteten Armen, wobei dieser gemessene Benutzer (8.2) in der Position mit ausgebreiteten Armen durch die Armstütze (3.4) und den Sitz (3.5) aufgenommen wird, die sich verlängern, drehen, umklappen und einstellen lassen.

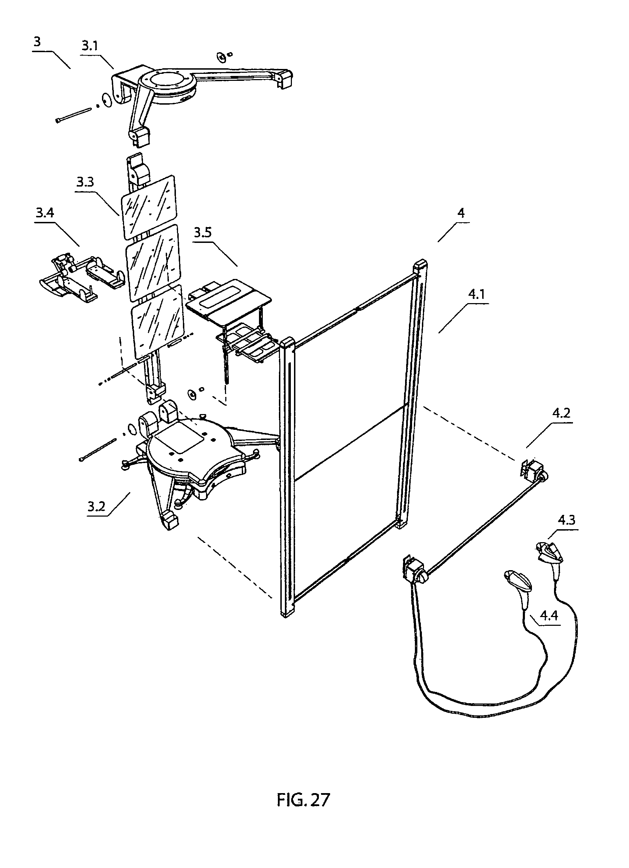

Figur 27. In dieser Figur ist eine auseinandergezogene perspektivische Vorderansicht der Hauptbestandteile auf auseinandergezogene Weise zu sehen.

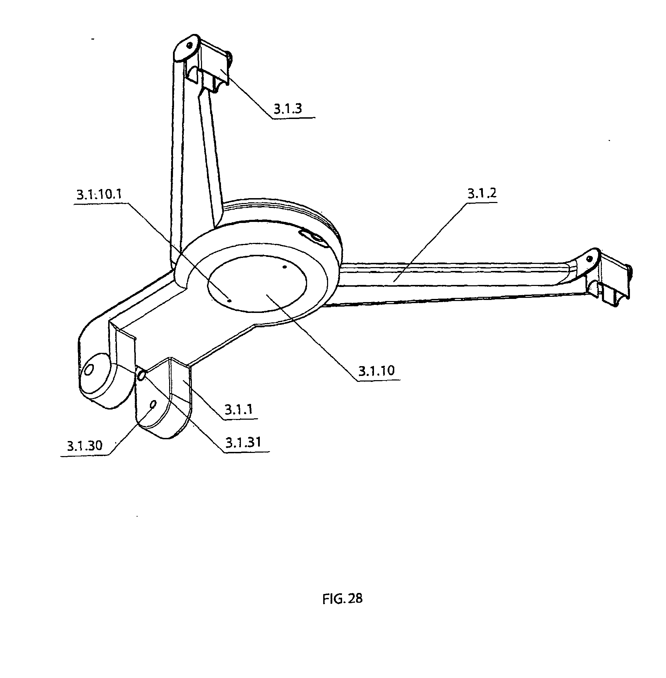

Figur 28. In dieser Figur ist eine obere perspektivische Vorderansicht der oberen Stützbaugruppe (3.1) zu sehen, wobei der Aufnahmerahmen B (3.1.2) einem anfangs zylindrischen Teil entspricht, der tangential eine Verlängerung ausstreckt, die an ihrem Endteil einen kleinen vielflächigen Teil ausspart. An den Innenflächen dieses ausgehöhlten Abschnitts ist ein kleiner laminarer Teil untergebracht, senkrecht zur Achse der bereits erwähnten Aufnahmeverlängerung A (3.1.3), die sich mittels eines zylindrischen Teils, des Aufnahmeverbinders (3.1.18) verbinden, der eine Pendelbewegung von (3.1.3) ermöglicht.

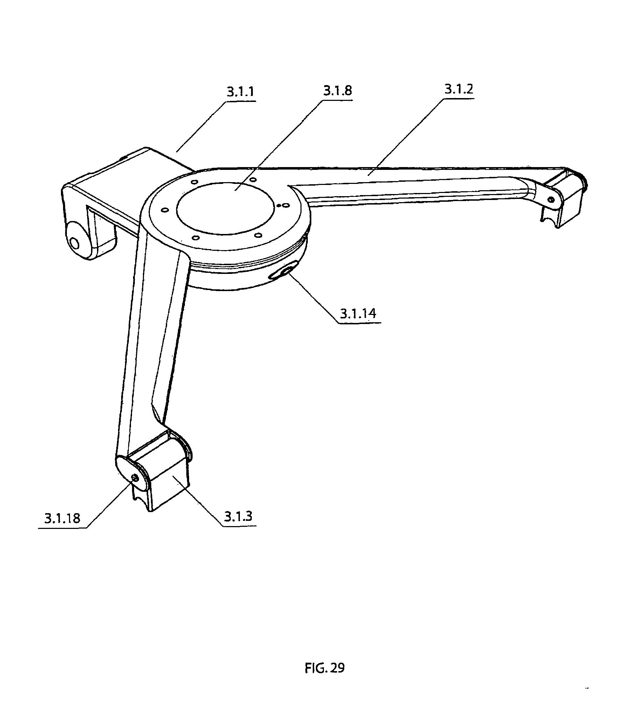

Figur 29. In dieser Figur ist eine untere perspektivische Vorderansicht der oberen Stützbaugruppe (3.1) zu sehen, wobei der große Teil (3.1.1) in seinem hinteren unteren Teil einen vielflächigen Abschnitt aufweist, der sich von seinem äußeren Seitenrand aus mit einer bestimmten Abmessung erstreckt, wobei sein Abschluss unter Bildung eines Halbzylinders erreicht wird, in dessen jeweiligem Mittelpunkt insgesamt eine Bohrung (3.1.30) hindurchgeht, außerdem ist eine Bohrung B mit einer bestimmten Tiefe nahe der vorherigen untergebracht. Dieser definierte Teil ist symmetrisch zur vertikalen Längsebene bei (3.1.1) aufgebaut.

Der Teil (3.1.10) weist in seinem äußeren Teil zwei Bohrungen (3.1.10.1), in gleichem Abstand vom jeweiligen Mittelpunkt, auf.

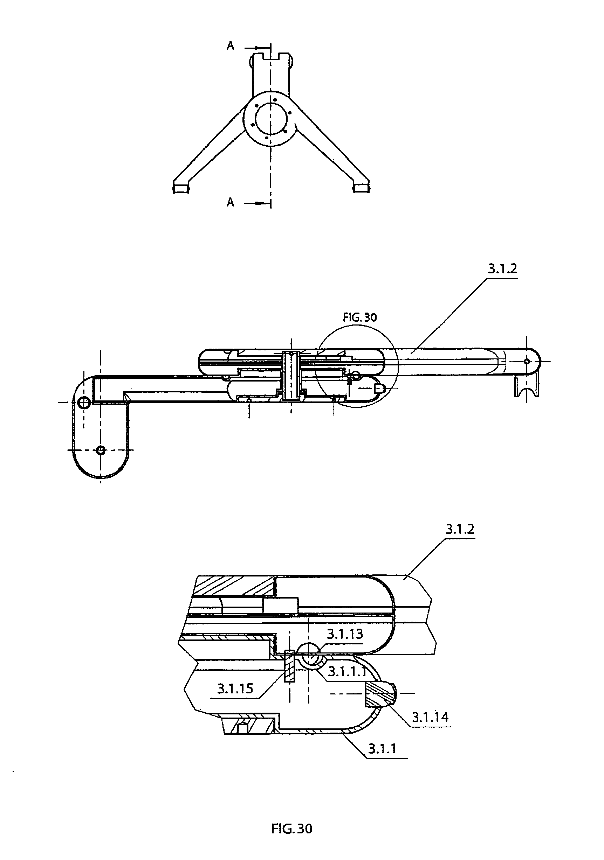

Figur 30. In dieser Figur ist ein Detail des Schnitts A der oberen Ansicht der oberen Stützbaugruppe (3.1) zu sehen, wobei die Basisstütze (3.1.1) einen durchgehenden Spalt (3.1.1.1) mit halbkreisförmigem Querschnitt aufweist, der einen sphärischen Teil (3.1.1.3) mit bestimmten Abmessungen und Eigenschaften aufnimmt, der in einen halbsphärischen Spalt in der Innenfläche von (3.1.2) aufnimmt, der mit gleichem Abstand und in einem gewissen Abstand vom jeweiligen Umkreismittelpunkt verteilt ist. Es ist ebenfalls ein zylindrisches Sicherungselement (3.1.15) zu erkennen, das in einer geräumigen Bohrung in dem ausgehöhlten Teil (3.1.2) untergebracht ist, die von dort aus senkrecht hindurchgeht, außerdem ermöglicht durch ihr Gegenstück in (3.1.1). Von dem letzteren aus erstreckt sich außerdem aus dessen ausgehöhltem Inneren und mittels einer Öffnung in dessen Vorderteil ein Ellipsoidstumpfteil, der Knopf (3.1.14).

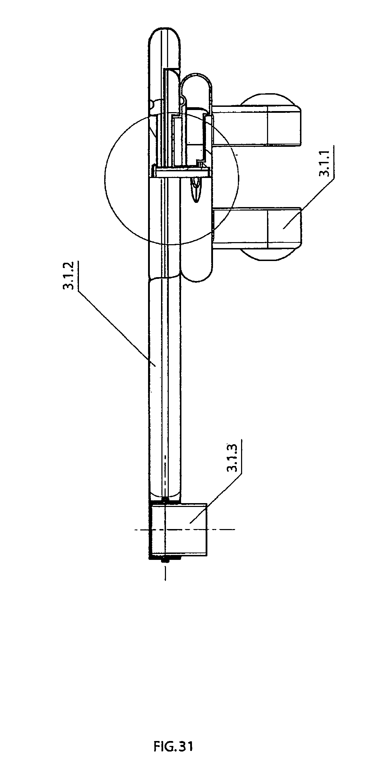

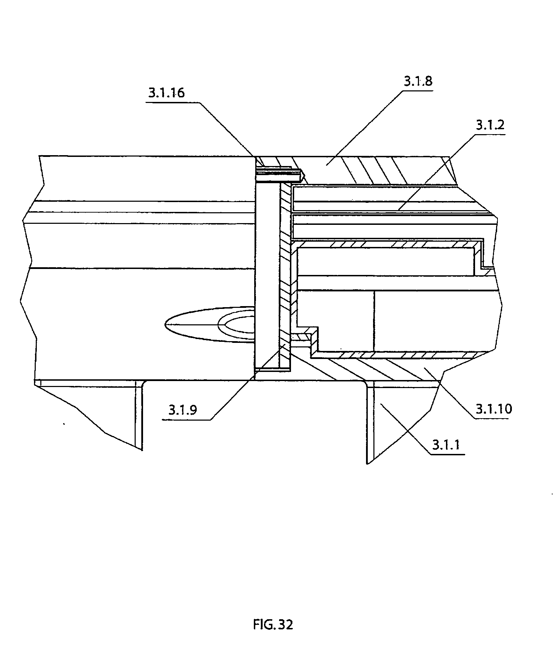

Figur 32. In dieser Figur ist ein Detail von Figur 31 zu sehen, eine aufgebrochene Ansicht in Vorderansicht der oberen Stützbaugruppe (3.1), wobei der große ausgehöhlte Körper, die Basisstütze (3.1.1) in einer Bohrung mit einem bestimmten Durchmesser mit dem Mittelpunkt im Ursprung ihrer Hauptkrümmung, den Aufnahmerahmen B (3.1.2) auf, der in seinem unteren Teil durch einen zylindrischen Teil mit einer bestimmten Dicke, die Aufnahmeachse A2 (3.1.10), und in seinem oberen Teil durch einen Teil mit einer bestimmten Dicke und einem bestimmten Durchmesser, die Aufnahmeachse A (3.1.8), verschlossen wird, wobei die zwei letzteren gemeinsame Endflächen haben, mit den Außenflächen in koplanarer Form zum Aufnahmerahmen B (3.1.2). Außerdem kreuzt ein kegelstumpfförmiger laminarer Teil (3.1.16) die Teile (3.1.8) und (3.1.9).

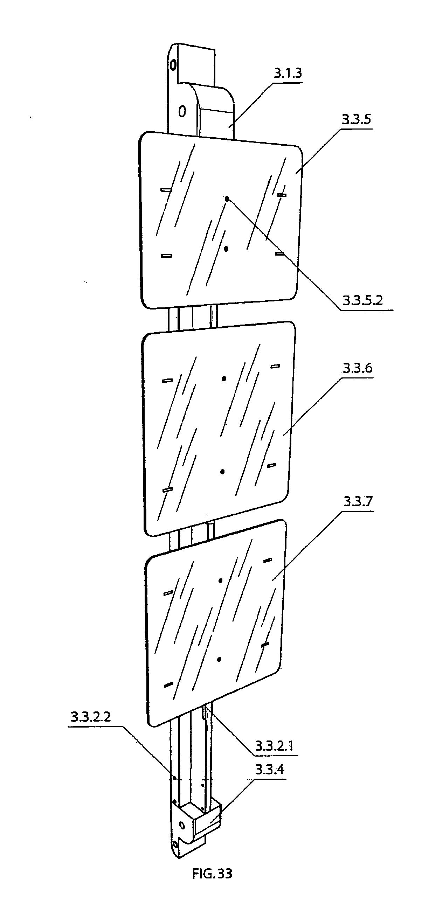

Figur 33. In dieser Figur ist eine perspektivische Vorderansicht der Säulenbaugruppe (3.3) zu sehen, wobei ein länglicher Teil (3.3.2.1) von dem ausgehöhlten Teil (3.3.2.2) ausgespart ist.

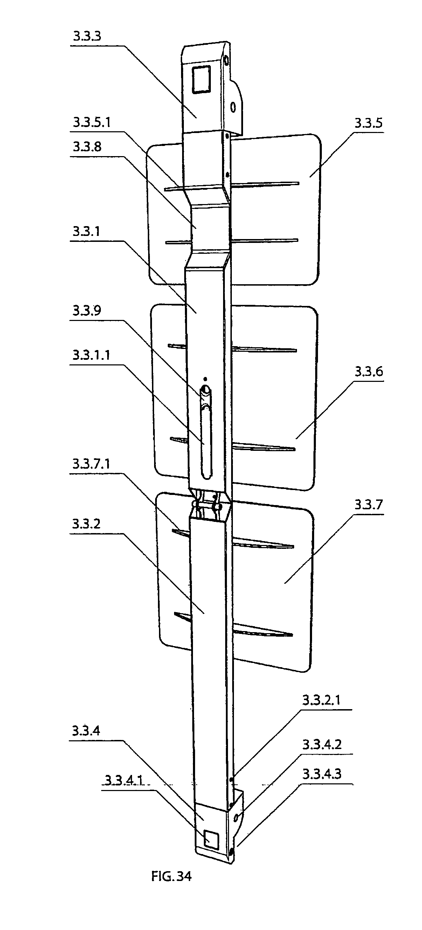

Figur 34. In dieser Figur ist eine perspektivische Rückansicht der Säulenbaugruppe (3.3) zu sehen, wobei ein ausgehöhlter Teil (3.3.2) mit Hilfe von Gewindeverbindern (3.3.2.1) mit einem vielflächigen ausgehöhlten Teil verbunden ist, der einen halbzylindrischen Abschnitt (3.3.4) aufweist. Der Teil (3.3.4) weist die Aussparung eines vielflächigen Teils auf, wobei sich darin und von dort aus bündig mit der Außenfläche ein Teil mit der besagten Form (3.3.4.1) erstreckt, tolerant für seine Bewegung in seiner vorgesehenen Achse, ebenso eine Rolle (3.3.4.3), auf beiden Seitenflächen und angenähert an die unterste Fläche, und eine Bohrung, die zu dem Teil hindurchgeht, im Hauptumkreismittelpunkt der Seitenfläche.

Zu erkennen ist ebenfalls ein laminarer Teil (3.3.7) mit einer bestimmten Dicke und mit einer rechteckigen Basis, die sich an die vordere Fläche von (3.3.2) anlehnt, von deren Anschluss ein weiterer laminarer Teil (3.3.7.1) angegliedert ist. Dieses Anlehnen wird dank zylindrischer und mit Gewinde versehener Teile (3.3.5.2.) senkrecht zur Hauptachse der Baugruppe erreicht (siehe in Figur 33).

Es ist die Aussparung eines Teils (3.3.1.1) zu erkennen, angeordnet in der hinteren Fläche des ausgehöhlten Teils (3.3.1), in dem, vorstehend aus dem Inneren, ein Teil (3.3.9) enthalten ist, der in seiner oberen und unteren Fläche halbzylindrische Vertiefungen aufweist, dazu in der Lage, sich in dem zuvor genannten zu verschieben. Dieser ausgehöhlte Teil (3.3.1) weist außerdem eine beträchtliche Mulde (3.3.8) nahe seinem äußersten oberen Rand auf.

Die besagten großen ausgehöhlten Teile (3.3.1 und 3.3.2) sind mittels zweier laminarer Teile (3.3.10 und sein symmetrisches Gegenstück) an beiden Innenseiten verbunden, die eine Bohrung aufweisen, die wiederum schließlich durch einen zylindrischen Teil (3.3.11) verbunden wird.

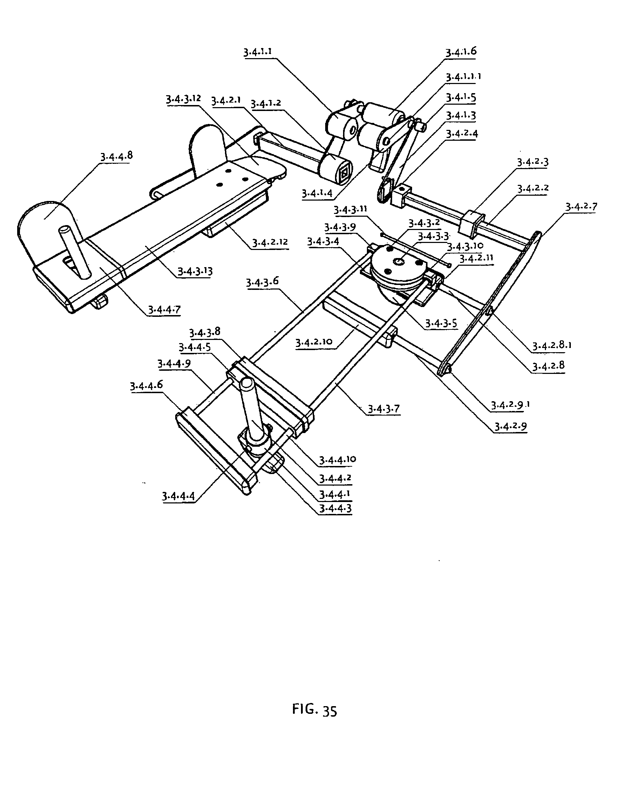

Figur 35. In dieser Figur ist eine perspektivische Vorderansicht der Armstützenbaugruppe (3.4) zu sehen, wobei ein zylindrischer Teil (3.4.1.1) verbunden ist mittels eines zylindrischen Teils mit kleinerem Durchmesser (3.4.1.1.1), der diesen und einen abfallenden laminaren Teil (3.4.1.4) kreuzt. Der letztere wiederum ist an seinem anderen Ende mit einem zylindrischen Teil (3.4.1.5) verbunden, der, mittels einer exzentrischen Bohrung, einen zylindrischen Teil (3.4.1.6) kreuzt, von dem aus sich in seinem mittleren unteren Abschnitt ein abfallender Teil mit gekrümmter Achse erstreckt, wobei sein Mittelpunkt zum Inneren der Baugruppe verschoben ist.

Gemeinsam mit (3.4.1.3) erstreckt sich in dessen Außenfläche ein ausgehöhlter Vielflächner (3.4.2.1), in dessen Innerem koaxial ein weiterer ähnlicher Teil mit kleinerer Abmessung (3.4.2.2) untergebracht ist, der mit einem Teil verbunden ist, der diesen Innenraum ausfüllt (3.4.2.4), seinerseits mittels eines zylindrischen Teils (3.4.2.5) verbunden. An den Teil (3.4.2.1) lehnt sich an dessen Außenrand ein Teil der den Abstand zwischen diesem und (3.4.2.2) ausfüllt, der Teil (3.4.2.3). An den Teil (3.4.2.2) schließt sich an seinem Außenrand ein großer laminarer Teil (3.4.2.7) an, senkrecht zu demselben und horizontal zur Baugruppe, von dem aus mittels von ausgerichteten Bohrungen zwei zylindrische Teile (3.4.2.9.1) und (3.4.2.8.1) zu sehen, welche die Teile (3.4.2.10) und (3.4.2.11) auf Abstand halten, in deren Zwischenraum zwei zylindrische röhrenförmige Teile (3.4.2.9) und (3.4.2.8) integriert sind. Die Teile (3.4.2.10) und (3.4.2.11) sind durch den Aufbau ausgerichtet und voneinander entfernt, wobei sie durch einen vielflächigen laminaren Teil (3.4.2.12) vereint sind, der an seiner Innenfläche eine Absenkung mit bestimmten Abmessungen aufweist, die in Vertikalrichtung einen Teil mit zylindrischer Basis aufnimmt, der sich nach unten erstreckt, mittels eines merklich größeren halbzylindrischen Teils (3.4.3.5). Über diesem erstreckt sich ein zylindrischer Teil (3.4.3.3), von dem aus er durch einen anderen Zylinder (3.4.3.1) mit einem merklich größeren Durchmesser befestigt ist, der an seinem hinteren Teil einen Einschnitt und an seiner oberen Fläche drei mit gleichem Abstand zu seiner Koaxialität angeordnete Bohrungen aufweist. In diesen Bohrungen sind drei zylindrische Teile (3.4.3.2) untergebracht, die es ermöglichen, einen großen laminaren Teil (3.4.3.13), von sehr nahe seinem hinteren Rand, zu verbinden. In der Öffnung seines anderen Endes steht aus seinem Inneren ein vielflächiger Teil (3.4.3.8) vor. An der Innenfläche desselben erstrecken sich zwei zylindrische Teile (3.4.3.6) und (3.4.3.7), an deren Ende sich zwei kleine vielflächige Teile (3.4.3.9) und (3.4.3.10) befinden.

Von den zylindrischen Teilen (3.4.3.6) und (3.4.3.7) schließen sich an ihrer End- und Außenfläche ein vielflächiger Teil (3.4.4.6) und in einem gewissen Abstand zum Inneren der Baugruppe ein Teil (3.4.4.5) an, der für diese Position zwei Bohrungen ausweist, welche die besagten zylindrischen Teile (3.4.3.6) und (3.4.3.7) hindurchlassen. Ein laminarer Teil (3.4.4.7) umhüllt die letzteren, der an seiner oberen und seiner unteren Fläche eine Vertiefung aufweist, die einen Teil aufnimmt, der sich nach oben erstreckt, wobei derselbe zylindrisch ist (3.4.4.10). Mit dem letzteren verbunden und im Inneren von (3.4.4.7) untergebracht befindet sich ein röhrenförmiger Teil (3.4.4.1), den ein Zylinder(3.4.4.4) mit kleinerem Durchmesser durchquert. An der Basis von (3.4.4.10) befindet sich, verbunden mittels eines inneren Fadens, ein Teil (3.4.4.3), ähnlich (3.4.3.5).

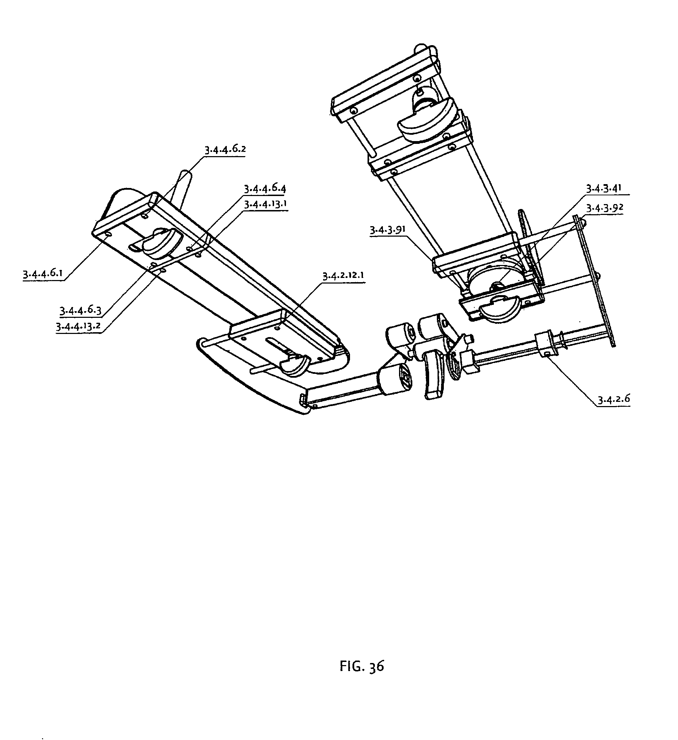

Figur 36. In dieser Figur ist eine perspektivische Unteransicht der Armstützenbaugruppe (3.4) zu sehen, wobei die zylindrischen mit Gewinde versehenen Teile (3.4.4.6.1), (3.4.4.6.2) (3.4.4.6.3) und (3.4.4.6.4) zu erkennen sind, die (3.4.4.7) positionieren. Auf gleiche Weise positionieren (3.4.3.13.1) und (3.4.3.13.2) (3.4.3.13).

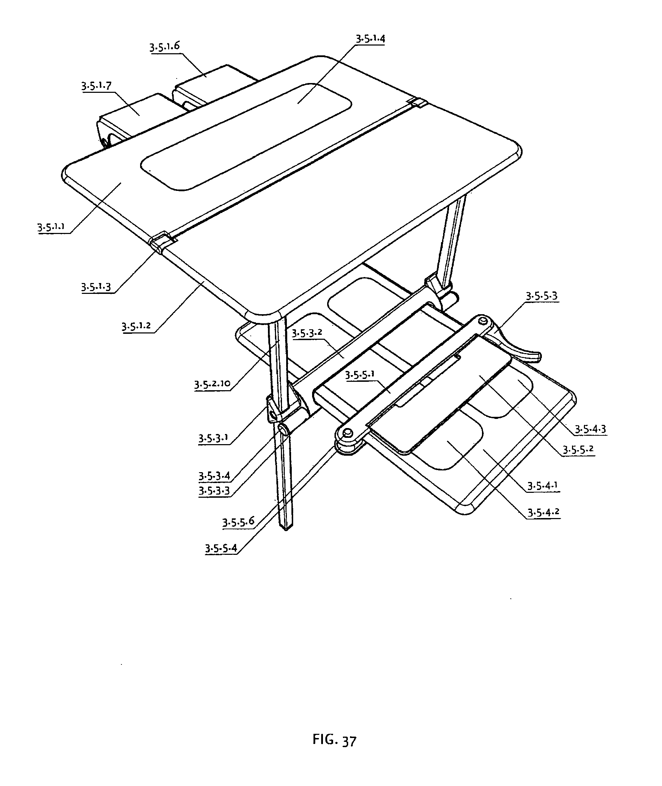

Figur 37. In dieser Figur ist eine perspektivische Vorderansicht der Sitzbaugruppe (3.5) zu sehen, wobei zwei große laminare Teile mit beträchtlicher Dicke (3.5.1.1) und (3.5.1.2) durch einen kleinen Teil (3.5.1.3) vereint sind. Ein angesetzter rechteckiger laminarer Teil (3.5.1.4) ungefähr in der Mitte von (3.5.1.1). Ein weiterer großer laminarer Teil (3.5.4.1) wird in einen länglichen Teil mit elliptischem Querschnitt (3.5.3.2) eingeführt, an dessen äußerster Fläche sich ein Teil mit dreieckigem Querschnitt (3.5.3.1) anschließt, der in Vertikalrichtung durch einen quadratischen röhrenförmigen Teil (3.5.2.10) durchquert wird. Über der seitlichen Außenfläche von (3.5.3.2) erstreckt sich ebenfalls ein zylindrischer Teil (3.5.3.3), der an seinem Ende gemeinsam mit einem merklich kleineren zylindrischen Teil (3.5.3.4) endet. Es ist ebenfalls ein länglicher Teil (3.5.5.6) mit rechteckiger Basis zu sehen, aus dessen Enden sich ein Halbzylinder formt, angeordnet quer über der oberen Fläche von (3.5.4.1), weist dieser einen zylindrischen Teil (3.5.5.6) auf, der ihn, an beiden Enden, in seinem jeweiligen Mittelpunkt durchquert, am rechten Ende, an der unteren Fläche des besagten Abschnitts, erstreckt sich ein Teil (3.5.5.4), und am linken Ende, ebenfalls im unteren Abschnitt, erstreckt sich ein Teil (3.5.5.3), dessen Achse eine doppelte Krümmung hat, wobei sich die radiale Mitte bei der zweiten und weiter außen gelegenen zum Äußeren der Baugruppe hin befindet. Über der oberen Fläche von (3.5.4.1) sind zwei parallele rechteckige Teile (3.5.4.2) und (3.5.4.3) angesetzt. Außerdem befindet sich in Teil (3.5.5.1) leicht eingesetzt ein laminarer Teil (3.5.5.2) mit bestimmten Abmessungen.

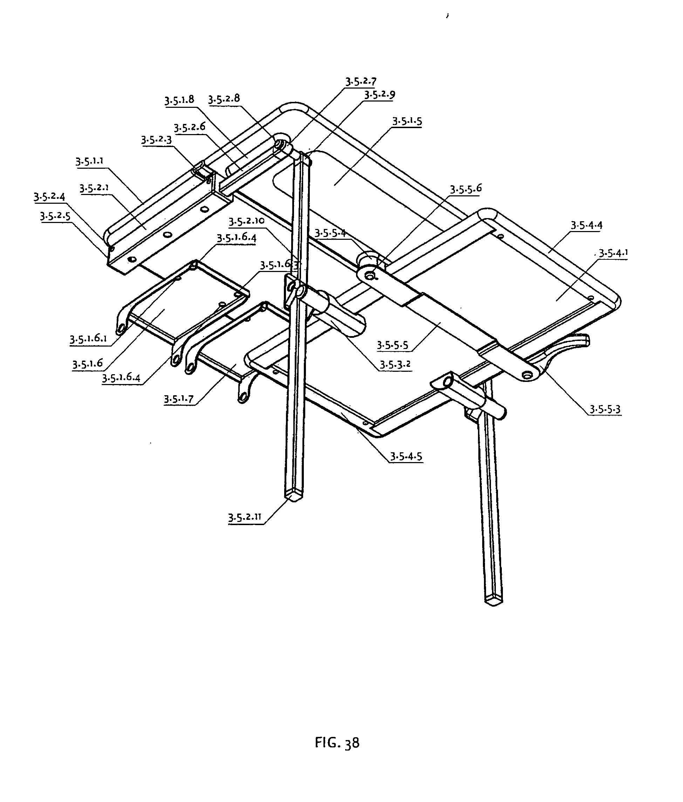

Figur 38. In dieser Figur ist eine untere perspektivische Ansicht der Sitzbaugruppe (3.5) zu sehen, wobei an der unteren und hinteren Fläche des Teils (3.5.1.1) parallel zum seitlichen Rand sich ein vielflächiger röhrenförmiger Teil (3.5.2.1) erstreckt, aus dessen Innerem und nach hinten sich ein Teil (3.5.2.5) erstreckt, nun erstreckt sich in seiner vorderen Öffnung ein vielflächiger röhrenförmiger Teil (3.5.2.2), aus dem ein weiterer vielflächiger röhrenförmiger Teil (3.5.2.6) mit in den Abmessungen kleinerem Querschnitt, vorsteht, wobei dieser an seinem vorderen Ende gemeinsam mit einem Teil mit halbzylindrischem Abschluss (3.5.2.7) vereint ist, das an seiner äußeren Seitenfläche mittels eines zylindrischen Teils (3.5.2.8) durchquert wird, der ihn mit einem entfernten ähnlichen Teil (3.5.2.9) verbindet. Von letzterem Teil nach unten erstreckt sich ein vielflächiger röhrenförmiger Teil (3.5.2.10) mit einer beträchtlichen Abmessung in seiner vertikalen Achse, der an seinem äußeren unteren Teil mit einem kleineren Teil (3.5.2.11) vereint ist.

Es ist außerdem am unteren Teil von (3.5.1.1) ein laminarer Teil (3.5.1.6) zu sehen, wobei mittels von zylindrischen Teilen (3.5.1.6.1), (3.5.1.6.2), (3.5.1.6.3) und (3.5.1.6.4) an seinem vorderen Abschnitt der äußere hintere Abschnitt mit dem hinteren Rand von (a1) verbunden ist. Wie können ebenfalls erkennen, dass der Teil (3.5.4.1) an seinen Enden durch (3.5.4.4) und (3.5.4.5) verschlossen ist, und einen nicht ebenen laminaren Teil (3.5.5.5), der (3.5.5.4) und (3.5.5.3) vereint.

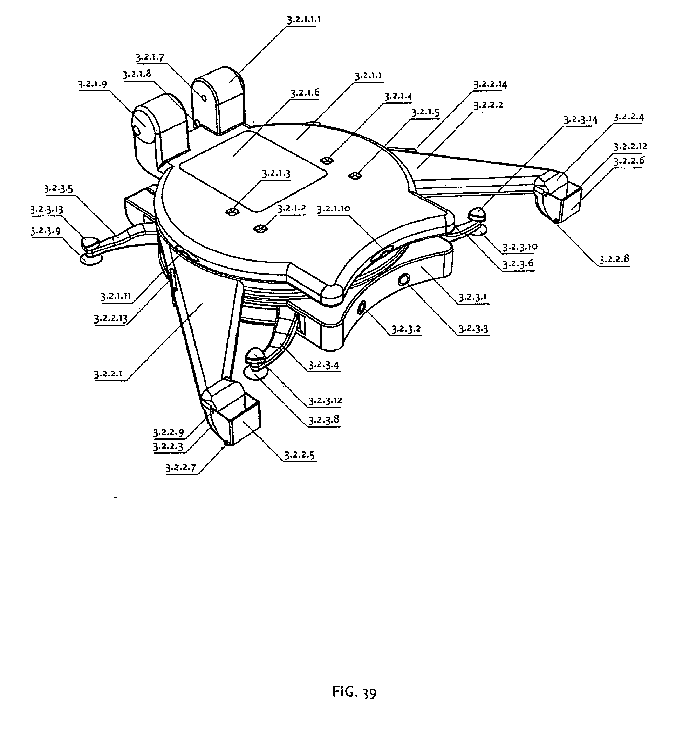

Figur 39. In dieser Figur ist eine perspektivische Vorderansicht der unteren Stützenbaugruppe(3.2) zu sehen, wobei ein ausgehöhlter Teil (3.2.1.1) mit einer im Wesentlichen kreisförmigen Basis sowohl an seinem vorderen als auch an seinem hinteren Teil eine rechteckige Verlängerung mit einer bestimmten Höhe (3.2.1.1) aufweist, wobei sich an seiner hinteren Oberfläche zwei parallele Teile (3.2.1.1.1) erstrecken und an seinem vorderen Abschnitt eine Rundhöhlung zu erkennen ist. Der besagte Teil (3.2.1.1.1) weist an seiner inneren Seitenfläche eine Bohrung (3.2.1.7) mit einer bestimmten Tiefe und über dieser eine Bohrung, die ihn durchquert (3.2.1.8), auf. Vier Mulden mit quadratischer Basis (3.2.1.2), (3.2.1.3), (3.2.1.4) und (3.2.1.5) sind über der oberen Fläche angeordnet, wobei sich zwei derselben in Längsrichtung ausgerichtet und nahe dem äußeren Rand befinden, während sich die anderen zwei symmetrisch zu denselben in einer Längsebene befinden. In der vorderen Rundhöhlung können wird einen ellipsoiden Teil (3.2.1.10) erkennen. An der konvexen Fläche können wir einen ähnlichen Teil (3.2.1.11) sehen.

Unter (3.2.1.1) können wir einen Teil (3.2.2.1) sehen, der sich tangential zur Zylindrizität von (3.2.1.1) verlängert, der abfällt und der sich in seinem äußeren Abschnitt mit einem Teil (3.2.2.3) vereint, der sich nach unten erstreckt, vorn umschlossen durch einen laminaren Teil (3.2.2.5), der an seiner seitlichen Basis einen zylindrischen Teil mit verringertem Durchmesser (3.2.2.7) aufweist, wobei es über der gleichen Fläche eine Bohrung (3.2.2.9) gibt.

Wiederum unter (3.2.2.1) können wir einen ausgehöhlten Teil (3.2.3.1), mit ähnlicher Basis wie (3.2.1.1), sehen, an dessen vorderem, ebenfalls konkavem, Teil sich zwei Spalte (3.2.3.2) und (3.2.3.3) befinden. An seiner Seitenfläche können wir einen großen Spalt sehen, aus dem ein abfallender Teil (3.2.3.4) mit gekrümmter Achse vorsteht, dessen Radiusmittelpunkt sich zum Inneren der Baugruppe hin befindet. Abschließend für diesen Teil und über seiner oberen Fläche befindet sich ein Teil mit dreieckiger Basis mit konvexen Seiten (3.2.3.12) und auf der anderen Seite desselben ein kegelstumpfförmiger Teil (3.2.3.8).

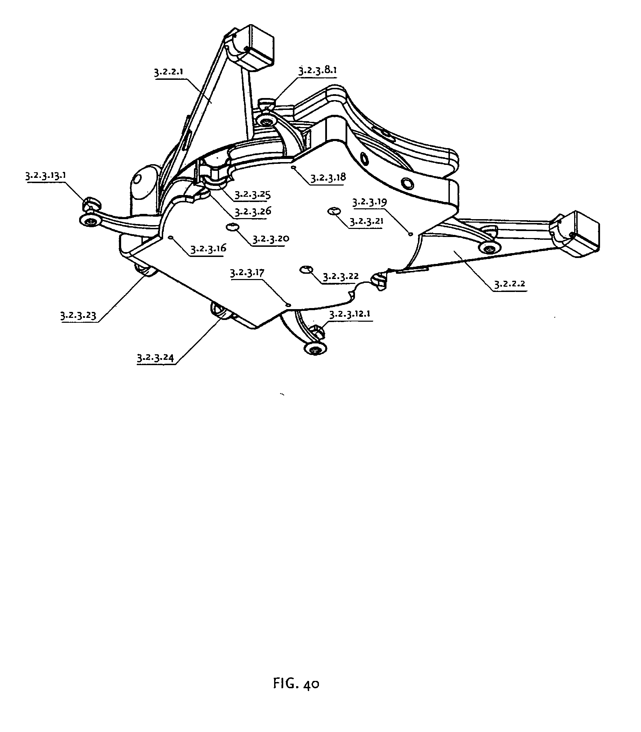

Figur 40. In dieser Figur ist eine untere perspektivische Ansicht der unteren Stützenbaugruppe (3.2) zu sehen, wobei es an der seitlichen und unteren Fläche des Teils (3.2.3.1) einen Spalt (3.2.3.25) und einen weiteren symmetrisch dazu (3.2.3.26) gibt; wir können ebenfalls drei große Bohrungen (3.2.3.20), (3.2.3.21) und (3.2.3.22) mit gleichem Abstand zur jeweiligen Koaxialität sehen, außerdem zwei kleine Bohrungen (3.2.3.16) und (3.2.3.18), die sich in symmetrischer Form (3.2.3.17) und (3.2.3.19) an der Längsebene wiederholen. Am hinteren Abschnitt können wir ein rechteckiges ausgespartes Volumen sehen, von dessen seitlichen Innenfläche ein zylindrischer Teil (3.2.3.24) vorsteht, der sich symmetrisch (3.2.3.23) an der besagten Ebene wiederholt.

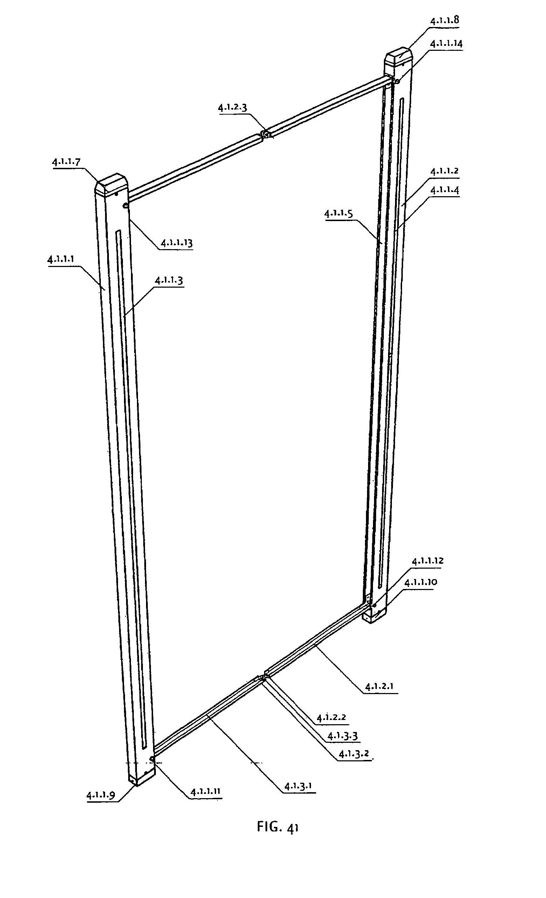

Figur 41. In dieser Figur ist eine perspektivische Vorderansicht der Rahmenbaugruppe (4.1) zu sehen, wobei ein röhrenförmiger Teil mit rechteckigem Querschnitt (4.1.1.1) an seiner vorderen Fläche eine durchgehende geradlinige Vertiefung (4.1.1.3) aufweist; über dieser gleichen Fläche sind an deren oberem Ende ein zylindrischer Teil (4.1.1.13) und an ihrem anderen Ende ein analoger Teil (4.1.1.11) zu sehen. An der inneren Seitenfläche des Teils (4.1.1.2) können wir eine große Vertiefung (4.1.1.5) erkennen; an ihrem äußersten unteren Abschnitt steht aus ihrem Inneren ein laminarer Teil (4.1.2.1) vor, offen an seinem oberen Teil, der gemeinsam mit einem laminaren Teil (4.1.2.2) vereint ist, der in einen ebenen Teil übergeht, wo ein Verbinder (4.1.3.3) untergebracht ist; von hier erstreckt sich ein Teil (4.1.3.2) symmetrisch zu (4.1.2.2) und angrenzend an einen Teil (4.1.3.1) symmetrisch zu (4.1.2.1). An der äußersten oberen Fläche von (4.1.1.1) befindet sich ein Prismenstumpfteil (4.1.1.7), und an seinem anderen Ende ein rechteckiger Teil (4.1.1.9).

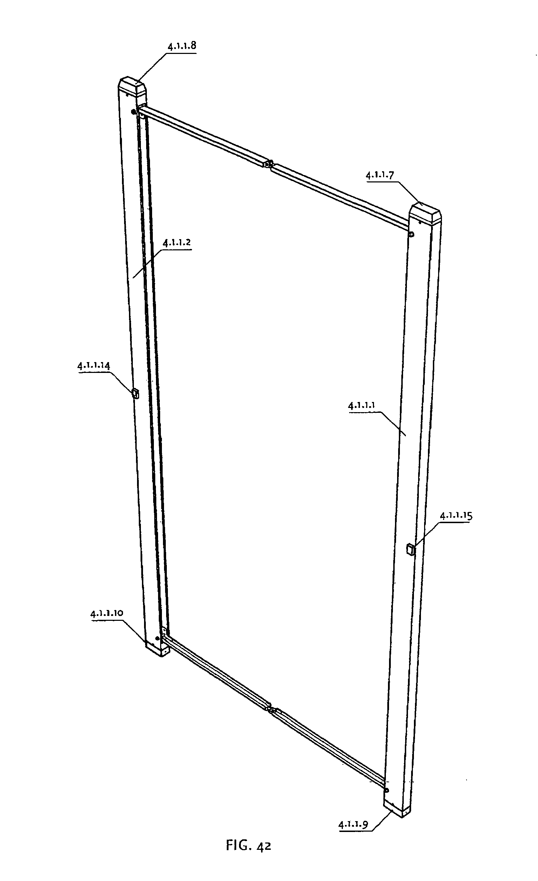

Figur 42. In dieser Figur ist eine perspektivische Rückansicht der Rahmenbaugruppe (4.1) zu sehen, wobei sich an der vorderen Fläche ein vielflächiger Teil (4.1.1.15) befindet, der aus dem Inneren derselben vorsteht; analog und symmetrisch zu demselben der vielflächige Teil (4.1.1.14).

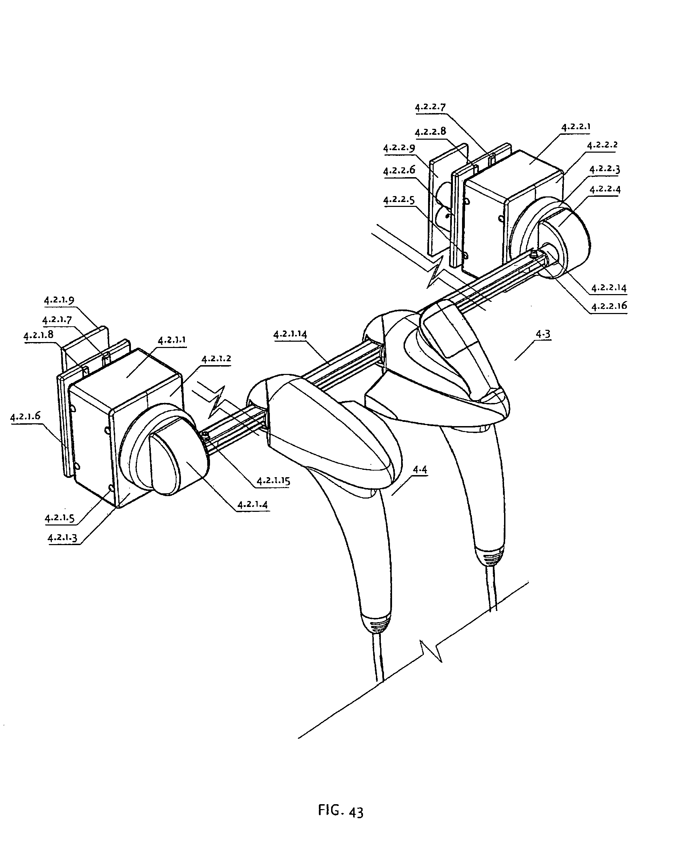

Figur 43. In dieser Figur ist eine perspektivische Vorderansicht der horizontalen Achsenbaugruppe (4.2) zu sehen, wobei ein ausgehöhlter vielflächiger Teil (4.2.1.1), an dessen vorderer Fläche sich mit dem gleichen Querschnitt ein Teil mit geringerer Dicke (4.2.1.2) erstreckt und sich ebenfalls von seiner vorderen Fläche ein zylindrischer Teil (4.2.1.3) mit erforderlichem Durchmesser erstreckt, von dessen vorderer Fläche wiederum sich ein Halbzylinder (4.2.1.4) erstreckt. Vom letzteren und von dessen seitlicher Innenfläche erstreckt sich ein röhrenförmiger Teil (4.2.1.14) mit einem vieleckigen Querschnitt, mittels eines zylindrischen Verbinders (4.2.1.15) der ihn durchquert. Am Ende von (4.2.1.14) ist ein kleiner zylindrischer Teil (4.2.2.14) angeschlossen, mittels eines ebenfalls zylindrischen Verbinders (4.2.1.16), der ihn durchquert; der besagte Teil (4.2.2.14) ist in den halbzylindrischen Teil (4.2.2.4) eingeführt, an dessen ebener Basis sich der zylindrische Teil (4.2.2.3) befindet, um auf einen vielflächigen Teil mit kleiner Dicke (4.2.2.2) zu treffen, an dessen hinterem Teil sich mit dem gleichen Querschnitt ein ausgehöhlter Teil (4.2.2.1) erstreckt. Hinter diesem können wir in einem gewissen Abstand einen laminaren Teil (4.2.2.5) erkennen, der zwei rechteckige magnetische Teile (4.2.2.7) und (4.2.2.8), mit merklich kleineren Abmessungen, enthält.

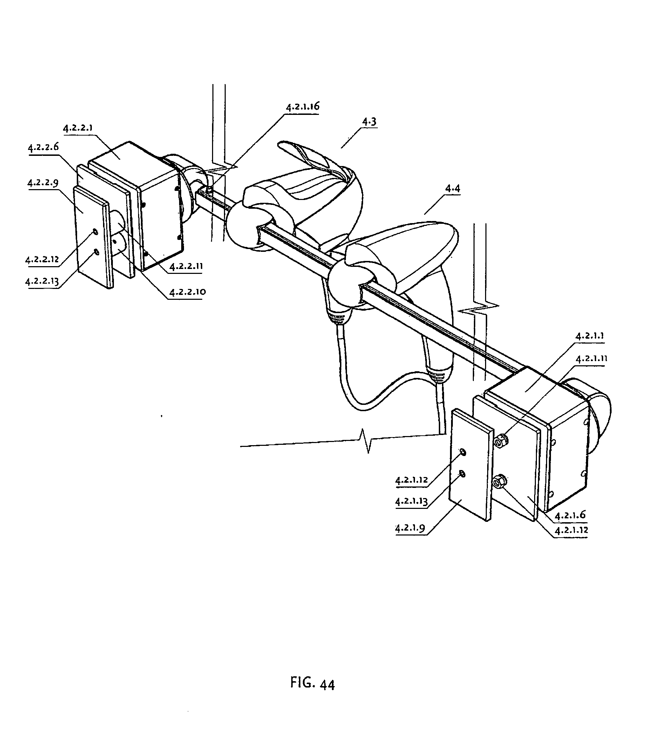

Figur 44. In dieser Figur ist eine perspektivische Rückansicht der horizontalen Achsenbaugruppe (4.2) zu sehen, wobei sich mit gleichem Abstand zum Teil (4.2.2.6) ein laminarer Teil (4.2.2.9) erstreckt, dessen Raum ein zylindrischer röhrenförmiger Teil (4.2.2.11) ausfüllt, und analog dazu (4.2.2.10).

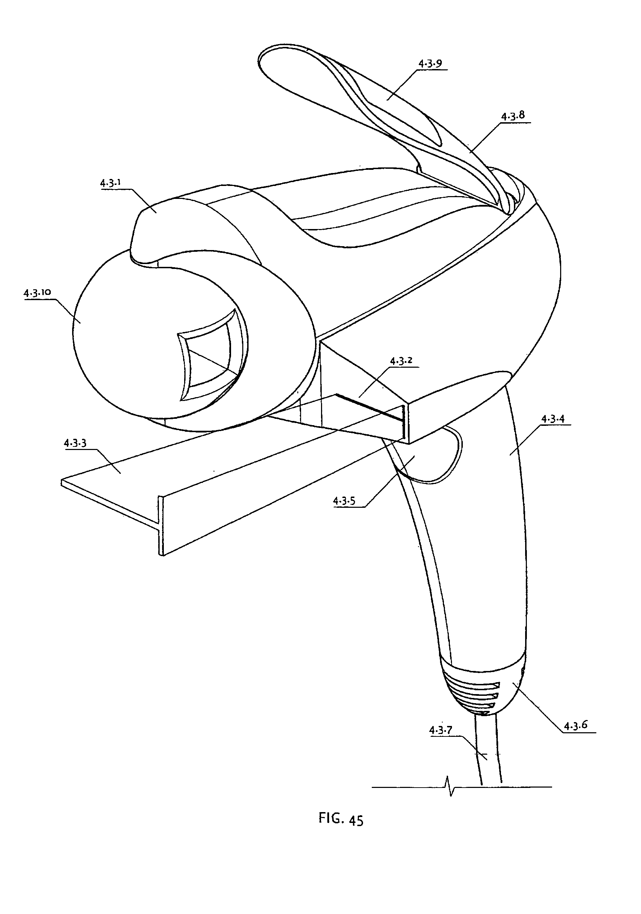

Figur 45. In dieser Figur ist eine perspektivische Vorderansicht der kartesischen Hauptmanipulator-Baugruppe (4.3) zu sehen, wobei ein großer ausgehöhlter Teil (4.3.1), ein Ellipsoid, in seinem vorderen Teil eine Rundhöhlung, gefolgt von einem Winkelschnitt in Bezug auf die Längsebene, aufweist, wovon sich außerdem ein kleiner Teil erstreckt, an dessen vorderer Fläche ein Teil (4.3.2) ähnlich einem umgedrehten "T" angesetzt ist, aus dem ein Lichtbündel (4.3.3) mit gleichem Querschnitt austritt. Unter dem (4.3.1) erstreckt sich gemeinsam ein abfallender Teil (4.3.4) mit gekrümmter Achse, wobei sich der Radiusmittelpunkt zum Vorderteil der Baugruppe hin befindet; an seinem Ende setzt ein Teil (4.3.6) fort, der einen abgerundeten Abschluss bietet; von dessen unterem Abschnitt erstreckt sich ein biegsamer zylindrischer Teil (4.3.7). An der Wölbung des Teils (4.3.1) ist ein sphärischer Teil (4.3.10) untergebracht, der quer zu der Baugruppe durch einen Teil mit vieleckigem Querschnitt ausgespart wird. An seinem hinteren oberen Teil ist ein gewundener Spalt zu sehen, wo sich ein gleichermaßen gewundener Teil (4.3.9) erstreckt, an dem ein kleiner vielflächiger Teil (4.3.8) angesetzt ist. Über dem Teil (4.3.4.) ist an dessen vorderem Abschnitt ein gewundener Teil (4.3.5) angesetzt.

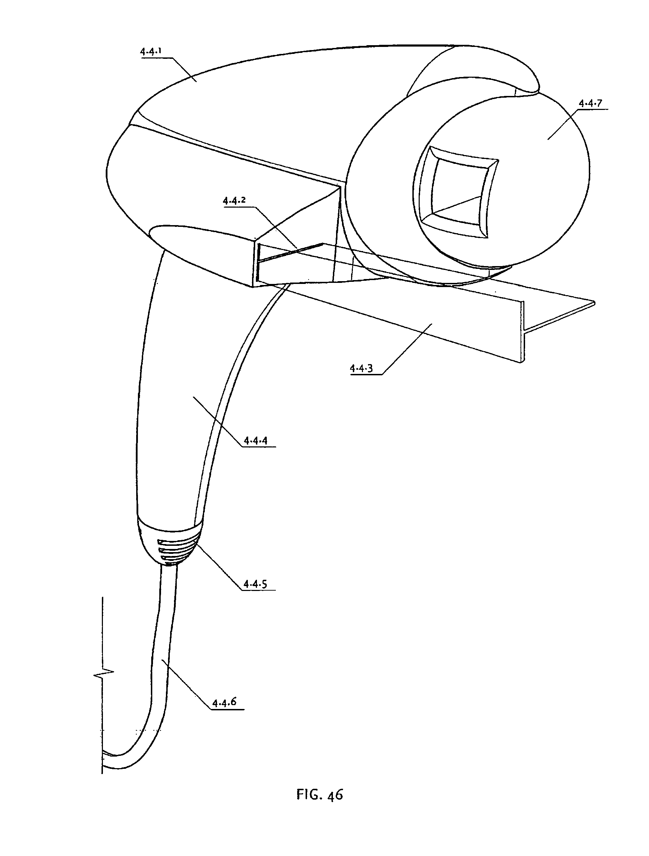

Figur 46. In dieser Figur ist eine perspektivische Vorderansicht der kartesischen Nebenmanipulator-Baugruppe (4.4) zu sehen, wobei es einen Teil (4.4.1), symmetrisch zu (4.3.1) gibt, und sich von dessen vorderer Oberfläche ein Teil (4.4.2), ebenfalls symmetrisch zu (4.3.2), erstreckt, von wo ein Lichtbündel (4.4.3) ausgeht.

Für Fachleute auf dem Gebiet wird es sich verstehen, dass sich das Vorstehende nur auf eine bevorzugte Ausführungsform der Erfindung bezieht, die empfänglich für Modifikationen ist, ohne dass dies bedeutet, vom Rahmen der Erfindung abzuweichen, wie er durch die folgenden Ansprüche definiert wird.

## Patentansprüche

1. Autonomes digitales transportables System zum Durchführen von anthropometrischen Messungen, das es ermöglicht, das Lokalisieren und Erfassen der anthropometrischen Punkte, die von dem zu messenden Individuum gewonnen werden zu erleichtern; Verfahren zum digitalisierten und automatisierten Speichern, Verarbeiten und Nachbearbeiten, selbiges in der Phase während der Messung (Fig. 15.1), **dadurch gekennzeichnet, dass** das System aus einem Haltungsausrichter (3), der das zu messende Individuum (8) aufnimmt, einen kartesischen Rahmen (4), der durch das messende Individuum (9) gehandhabt wird, ein Rechneranwendungsprogramm (5), ein Notebook oder ähnliches (5.1), drahtgebundene oder drahtlose Gebergeräte (6 und 7), wobei der kartesische Rahmen (4), insbesondere die kartesischen Manipulatoren (4,4 und 4,3) ein Lichtbündel (4.3.3) aussenden, das sich zu dem zu messenden Individuum (8) hin erstreckt, dafür vorgesehen, auf rechtwinklige und schräge Weise einen Bezug für die Lokalisierung des erforderlichen anthropometrischen Punktes herzustellen, wobei diese durch die in den kartesischen Manipulatoren (4,4 und 4,3) untergebrachten Sensoren verarbeiteten Informationen mittels einer schwenkbaren Anzeige (4.3.9) vorvisualisiert werden, wobei diese anthropometrischen Abmessungen digital in dem in einem vertikalen großen ausgehöhlten Teil (4.2.1.1) untergebrachten Prozessor gespeichert, während oder nach der Durchführung der Messung über drahtgebundene oder drahtlose Gebergeräte (6) an das in einem Notebook oder ähnlichem (5.1) installierte Rechneranwendungsprogramm (5) übertragen werden, wobei Übertragungsfehler vermieden werden, neben der Verbesserung der Visualisierung der Daten und der Verringerung der Arbeitszeit der Messung.

2. System nach Anspruch 1, **dadurch gekennzeichnet, dass** der Haltungsausrichter (3)aus einer oberen Stütze (3.1), einer unteren Stütze (3.2) und einer Säule (3.3) besteht, wobei die letztere dieselben vereint und gelenkig verbindet, wobei sie ihrerseits aus einer Armstütze (3.4) und einem Sitz (3,5) gebildet wird, wobei diese Baugruppe ermöglicht, die Haltungen des zu messenden Individuums (8) aufzunehmen und es von dem physischen Ort zu isolieren, wo die Messung vorgenommen werden soll.

3. System nach Anspruch 2, **dadurch gekennzeichnet, dass** der Haltungsausrichter (3) außerdem, wenn sich der gemessene Benutzer (8) auf der unteren Stütze (3.2) befindet, das Gewicht des Individuums erfasst, um es mit Kabel-Gebergeräten (7) an das in dem Notebook oder ähnlichem (5.1) installierte Rechneranwendungsprogramm (5) weiterzuleiten, wobei vermieden wird, dass Hilfselemente benutzt werden, die den Messvorgang dehnen.

4. System nach Anspruch 2, **dadurch gekennzeichnet, dass** die obere Stütze (3.1) eine umklappbare, in der Aufnahmedrehung abweichend positionseingeschränkte Stütze ist.

5. System nach Anspruch 2, **dadurch gekennzeichnet, dass** die untere Stütze (3.2) eine umklappbare, in der Aufnahmedrehung abweichend auf vier Positionen eingeschränkte Behälterstütze ist.

6. Vorrichtung nach Anspruch 2, **dadurch gekennzeichnet, dass** die Säule (3.3) eine schwenkbare Säule mit zwei umklappbaren Wirbeln ist, die sich mittels ihrer Platten und Ebenen anpassen.

7. Vorrichtung nach Anspruch 2, **dadurch gekennzeichnet, dass** die Armstütze (3.4) eine Stütze für die oberen Gliedmaßen sowohl eines Kindes als auch für Erwachsene ist, in mehreren Stellungen in Ausdehnung und Drehung regulierbar.

8. Vorrichtung nach Anspruch 2, **dadurch gekennzeichnet, dass** der Sitz (3.5) ein Sitz sowohl eines Kindes als auch für Erwachsene ist, in mehreren Stellungen in Ausdehnung und umklappbar regulierbar, wobei das Individuum eine Sitzposition einnimmt und sich die Vorrichtungen dieser Haltung anpassen.

9. Vorrichtung nach Anspruch 1, **dadurch gekennzeichnet, dass** der kartesische Rahmen (4) aus einem Rahmen (4.1) und einer horizontalen Achse (4.2) besteht, wobei der erstere das System strukturiert und die zweite sich in paralleler Form in einer Axialrichtung über den ersteren verschiebt, wobei diese Baugruppe eine unmittelbare Beziehung zu dem messenden Benutzer (9) hat, wobei die instrumentelle Instabilität vermieden wird, was die Messung genauer und zuverlässiger macht, wobei sie außerdem dazu in der Lage ist, das Lokalisieren von zwei Punkten auf aufeinanderfolgende Weise zu erleichtern und sie unter Umständen auf simultane Weise aufzuzeichnen.

10. Vorrichtung nach Anspruch 4, **dadurch gekennzeichnet, dass** die Baugruppe (4.2) mittels des gestischen Eingreifens des messenden Benutzers (9) leicht in einer Hauptachsenrichtung in Bezug auf (4) verschoben werden kann, so dass von einer kartesischen Ebene aus in einer Richtung "Y" die Stabilität, Zuverlässigkeit und folglich Genauigkeit und Verringerung der Fehlerhäufigkeit der Messung gesichert wird.

11. Vorrichtung nach Anspruch 4, **dadurch gekennzeichnet, dass** die kartesischen Manipulatoren (4.2.3 und 4.2.4) mittels des gestischen Eingreifens des messenden Benutzers (9) leicht in einer Hauptachsenrichtung in Bezug auf (4.2.1.14) unabhängig und leicht verschoben werden können, so dass von einer kartesischen Ebene aus in einer Richtung "X" die Stabilität, Zuverlässigkeit und folglich Genauigkeit und Verringerung der Fehlerhäufigkeit der Messung gesichert wird.

12. Vorrichtung nach Anspruch 4, **dadurch gekennzeichnet, dass** der Rahmen (4.1) eine zu entfaltende abweichend parallele Behälterstütze ist.

13. Vorrichtung nach Anspruch 4, **dadurch gekennzeichnet, dass** die horizontale Achse (4.2) eine in X verschiebbare und in (4.2.1.3) drehbare Behälterstütze ist.

14. Vorrichtung nach Anspruch 4, **dadurch gekennzeichnet, dass** der kartesische Hauptmanipulator (4.3) ein Behälter ist, der verschoben und gedreht werden kann und der ein sichtbares Strahlenbündel (4.3.3) ausstrahlt, wobei er einen Teil (4.3.8) aufweist, der zum Hauptteil geschwenkt werden kann, der die Abmessungen sichtbar macht, wobei der Hauptteil, in dem die Sensoren untergebracht sind, welche die Messung vornehmen, aus einem greifbaren Teil (4.3.4) besteht, in dem sich ein zu drückender Teil (4.3.5) befindet, wobei mit dieser Baugruppe das Lokalisieren des ersten anthropometrischen Punktes und das Sichtbarmachen der Abmessungen, die von demselben ausgezeichnet werden, des Gewichts, der Vibration, der Winkel und dessen, der im Folgenden beschrieben wird, erleichtert wird.

15. Vorrichtung nach Anspruch 4, **dadurch gekennzeichnet, dass** der kartesische Nebenmanipulator (4.4) ein Behälter ist, der verschoben und gedreht werden kann und der ein sichtbares Strahlenbündel (4.3.4) ausstrahlt, wobei dies ermöglicht, den zweiten anthropometrischen Punkt zu lokalisieren, erleichtert durch das ausgestrahlte sichtbare Strahlenbündel (4.3.3).

16. Vorrichtung nach Anspruch 1, **dadurch gekennzeichnet, dass** sich der kartesische Rahmen (4) um den Haltungsausrichter (3) dreht, wobei dies die stabile Messung des zu messenden Benutzers (8) in seinen vier Hauptprojektionsansichten ermöglicht, der Vorderansicht, der rechten Seitenansicht, der Rückansicht und der linken Seitenansicht, wobei vermieden wird, das Individuum in einer der gewünschten Haltungen zu bewegen.

17. Vorrichtung nach Anspruch 1, **dadurch gekennzeichnet, dass** der Haltungsausrichter (3) die anthropometrische Betrachtungs- oder stehende Haltung des Individuums (8) mittels transparenter laminarer Stützen (3.3) aufnehmen kann, wobei der Stauzustand der Armstütze (3.4) und die Koplanarität des Stauzustandes des Sitzes (3,5) das erforderliche Haltungsprotokoll sichern.

18. Vorrichtung nach Anspruch 1, **dadurch gekennzeichnet, dass** der Haltungsausrichter (3) die modifizierte anthropometrische Betrachtungs- oder sitzende Haltung des Individuums (8.1) mittels transparenter laminarer Stützen (3.3) aufnehmen kann, wobei die Einstellbarkeit und die mehrfachen Haltungen, welche die Armstütze (3.4) bietet, ebenso die Einstellbarkeit und die mehrfachen Haltungen, welche der Sitz (3,5) bietet, das erforderliche Haltungsprotokoll sichern.

19. Vorrichtung nach Anspruch 1, **dadurch gekennzeichnet, dass** der Haltungsausrichter (3) die Haltung des Individuums (8.1) mit ausgebreiteten Armen mittels transparenter laminarer Stützen (3.3) aufnehmen kann, die Einstellbarkeit und die mehrfachen Haltungen, welche die Armstütze (3.4) bietet, und die Koplanarität des Stauzustandes des Sitzes (3,5) das erforderliche Haltungsprotokoll sichern.

20. Vorrichtung in ihrer Phase vor dem Durchführen der Messung (Fig. 2 und Fig. 3), bestehend aus dem Haltungsausrichter (3) und dem kartesischen Rahmen (4), die entsprechende Schutzvorrichtungen, für (3) die Schutzvorrichtung (1) und für (4) die Schutzvorrichtung (2), aufweisen, **dadurch gekennzeichnet, dass** der Haltungsausrichter (3) und der kartesische Rahmen (4) einzeln in ihren bereits erwähnten Schutzvorrichtungen transportiert werden, was ermöglicht, dass sie von Individuen in vertikalen Höhen verschoben und auf dem Bodenniveau umgesetzt und durch Fahrzeuge mit mittlerem Fassungsvermögen transportiert werden, wobei vermieden wird, dass ihre für Beschädigungen anfälligen Hauptbestandteile vor Stößen geschützt sind, also ermöglicht, dass das Instrumentarium als zwei komplementäre Einheiten vor Ort gebracht werden und messen kann.

21. Vorrichtung nach Anspruch 20, **dadurch gekennzeichnet, dass** bei der Haltungsausrichtereinheit (3) ihr Selbstschutz mittels des Faltens, der Einziehbarkeit, der Gelenkigkeit und der Einstallung ihrer Hauptbestandteile berücksichtigt ist, wodurch der Verlust derselben vermieden wird.

22. Vorrichtung nach Anspruch 20, **dadurch gekennzeichnet, dass** bei der Stützeinheit (4) ihr Selbstschutz mittels des Faltens, der Einziehbarkeit, der Gelenkigkeit und der Einstallung ihrer Hauptbestandteile berücksichtigt ist, wodurch der Verlust derselben vermieden wird und die diesbezüglichen Arbeitsgänge und Zeiten verringert werden.

23. Vorrichtung in ihrer Phase des Koppelns (Fig. 14), bestehend aus den komplementären Einheiten, dem Haltungsausrichter (3) und dem kartesischen Rahmen (4), **dadurch gekennzeichnet**, das der kartesische Rahmen (4) an den Haltungsausrichter (3) gekoppelt wird, wobei dies **dadurch** bewirkt wird, dass zuerst, geführt durch ein Individuum, das der messende Benutzer (9) sein kann, (4.1.1) eingesetzt wird, danach (4.1.2) in (3.2.2.3) angeschlossen und mit (3.2.2) befestigt wird, wobei so mittels komplementärer Einheiten ermöglicht wird, eine Einheit zu bilden, welche die 37 anthropometrischen Maße umsetzt, und weitere Zubehörteile zu vermeiden.

24. Vorrichtung nach Anspruch 1, bestehend aus den komplementären Einheiten, der Stütze (3) und dem kartesischen Rahmen (4), und **dadurch gekennzeichnet**, das der kartesische Rahmen (4) und der Haltungsausrichter (3) mittels der Geber (6 und 7) digitale Daten an die in dem Notebook oder ähnlichem (5.1) installierte Software (5) übergeben, wobei dies **dadurch** bewirkt wird, dass zuerst ein Geber (7) in den ausgehöhlten Körper (4.2.5) und danach der Geber 86) in die Basis (3.2.1) eingesetzt wird, wobei die Reihenfolge des Vorstehenden nicht wichtig ist, wobei dies durch den messenden Benutzer (9) bewirkt werden kann, wobei so mittels komplementärer Einheiten ermöglicht wird, eine Einheit zu bilden, die von der Software unterstützt wird, wobei die anthropometrischen Daten in digitaler und automatischer Form gespeichert, verarbeitet und nachbearbeitet werden.

25. Vorrichtung nach Anspruch 24, bestehend aus den komplementären Einheiten, dem Haltungsausrichter (3) und dem kartesischen Rahmen (4), den Gebern (6 und 7) für die in dem Notebook oder ähnlichem (5.19 installierte Software (5), und **dadurch gekennzeichnet dass** die Unterstützung der Software es ermöglicht, die Einheit (3 und 4) zu nivellieren, wobei die zu entfaltenden und regulierenden Füße (3.2.3.12) entsprechend eingestellt werden.

26. Gestisch-operatives Verfahren zur Mustermessung nach Anspruch 1, **dadurch gekennzeichnet, dass**:
a) der messende Benutzer (9) manuell und taktil den jeweiligen anthropometrischen Punkt lokalisieren muss, der zu dem gemessenen Benutzer (8) gehört, der dem Protokoll gefolgt ist und die entsprechende anthropometrische Haltung einnimmt, danach
b) den Nebenmanipulator (4.4) diesem Punkt entgegen verschieben muss, geführt durch das von diesem zum Körper des gemessenen Benutzers (8) hin ausgestrahlte Lichtbündel, wobei dieses als Anfangspunkt der Entfernung zu verstehen ist, hiernach
c) der folgende anthropometrische Punkt, genauso wie der erste, geortet werden muss,
d) die horizontale Achsenbaugruppe (4.2) bewegen, falls es erforderlich ist, danach
e) der Hauptmanipulator (4.3) , genauso wie in b), dem folgenden anthropometrischen Punkt entgegen verschoben werden muss, schließlich
f) machen Sie das Maß in der schwenkbaren Voranzeige (4.3.8) sichtbar und geben Sie, mittels Druck des Steuergeräts (4.3.5), falls es erforderlich ist, die digitalen Daten ein,
g) setzen Sie, indem Sie zweimal das Steuergerät (4.3.5) drücken, den aufgezeichneten Wert zurück und löschen Sie ihn, wobei das Verfahren das Verhältnis von Zeit und operativen Gesten verbessert, indem es in flinke, stabile und genauere Manöver aufgelöst wird, so gibt es ein Instrument, das Abmessungsskalenverhältnis verbessert, wobei ein Spezialist, der messende Benutzer (9), mit seiner Wahrnehmung und seinem Urteilsvermögen die verschiedenen anthropometrischen Punkte des gemessenen Benutzers (8) aufnimmt.

27. Gestisch-operatives Verfahren zur horizontalen schrägen Messung nach Anspruch 1, **dadurch gekennzeichnet, dass**:
a) der messende Benutzer (9) manuell und taktil den jeweiligen anthropometrischen Punkt lokalisieren muss, der zu dem gemessenen Benutzer (8) gehört, der dem Protokoll gefolgt ist und die entsprechende anthropometrische Haltung mit ausgebreiteten Armen einnimmt, danach
b) den Nebenmanipulator (4.2.4) diesem Punkt entgegen verschieben muss, geführt durch das von diesem zum Körper des gemessenen Benutzers (8) hin ausgestrahlte Lichtbündel, wobei dieses als Anfangspunkt der Entfernung zu verstehen ist, hiernach
c) muss in dem Fall, dass der gemessene Benutzer (8) die horizontale Reichweite der Baugruppe (4.2.) überschreitet, der Nebenmanipulator (4.4) dem entsprechenden Punkt entgegen gedreht werden, geführt durch das von diesem zum Körper des gemessenen Benutzers (8) hin ausgestrahlte Lichtbündel, wobei der Winkel Alpha gebildet wird,
d) der folgende anthropometrische Punkt, genauso wie der erste, geortet werden muss,
e) die horizontale Achsenbaugruppe (4.2) bewegen, falls es erforderlich ist, danach
f) der Hauptmanipulator (4.3), genauso wie in b), dem folgenden anthropometrischen Punkt entgegen verschoben werden muss,
g) falls es erforderlich ist, verfahren Sie symmetrisch zu c), wobei der Winkel Beta gebildet wird, schließlich
h) machen Sie das Maß in der schwenkbaren Voranzeige (4.3.8) sichtbar und geben Sie, mittels Druck des Steuergeräts (4.3.5), falls es erforderlich ist, die digitalen Daten ein,
i) setzen Sie, indem Sie zweimal das Steuergerät (4.3.5) drücken, den aufgezeichneten Wert zurück und löschen Sie ihn, so gibt es ein Verfahren, das durch die Vorrichtung erleichtert wird, die es ermöglicht, Messungen aufzuzeichnen, welche die jeweiligen Reichweiten des Instruments überschreiten, wobei eine solche Lösung die instrumentelle, operative und gestische Stabilität sowie die Verringerung der dafür erforderlichen Zeit verbessert.

28. Gestisch-operatives Verfahren zur vertikalen schrägen Messung nach Anspruch 1, **dadurch gekennzeichnet, dass**:
a) der messende Benutzer (9) manuell und taktil den jeweiligen anthropometrischen Punkt lokalisieren muss, der zu dem gemessenen Benutzer (8) gehört, der dem Protokoll gefolgt ist und die entsprechende anthropometrische Haltung einnimmt, danach
b) den Nebenmanipulator (4.4) diesem Punkt entgegen verschieben muss, geführt durch das von diesem zum Körper des gemessenen Benutzers (8) hin ausgestrahlte Lichtbündel, wobei dieses als Anfangspunkt der Entfernung zu verstehen ist, hiernach
c) muss in dem Fall, dass die über den gemessenen Benutzer (8) zu messende Abmessung durch ein eigenes Körpersegment des Betreffenden unterbrochen wird, wird die horizontale Achsenbaugruppe (4.2) gedreht werden müssen, die schwenkt und sich in (4.2.2.1) dem entsprechenden Punkt entgegen losmacht, geführt durch das von diesem zum Körper des gemessenen Benutzers (8) hin ausgestrahlte Lichtbündel, wobei der Winkel Beta gebildet wird,
d) der folgende anthropometrische Punkt, genauso wie der erste, geortet werden muss,
e) die horizontale Achsenbaugruppe (4.2) bewegen, falls es erforderlich ist, danach
f) der Hauptmanipulator (4.3), genauso wie in b), dem folgenden anthropometrischen Punkt entgegen verschoben werden muss,
g) machen Sie das Maß in der schwenkbaren Voranzeige (4.3.8) sichtbar und geben Sie, mittels Druck des Steuergeräts (4.3.5), falls es erforderlich ist, die digitalen Daten ein,
h) setzen Sie, indem Sie zweimal das Steuergerät (4.3.5) drücken, den aufgezeichneten Wert zurück und löschen Sie ihn, so gibt es ein Verfahren, das durch die Vorrichtung erleichtert wird, die es ermöglicht, Messungen aufzuzeichnen, die durch die eigene Haltungs- sowie Körperbeschaffenheit des gemessenen Benutzers (8) unterbrochen werden, wobei eine solche Lösung die instrumentelle, operative und gestische Stabilität, sowie die Verringerung der dafür erforderlichen Zeit verbessert.

29. Gestisch-operatives Verfahren zur horizontalen ausgerichteten Messung nach Anspruch 1, **dadurch gekennzeichnet, dass**:
a) die Sicherung (4.2.10) losgemacht werden muss,
b) der messende Benutzer (9) manuell und taktil den jeweiligen anthropometrischen Punkt lokalisieren muss, der zu dem gemessenen Benutzer (8) gehört, der als Beispiel (Fig. 23), in einer sitzenden Haltung, seine obere Gliedmaße ausgestreckt und angehoben hat, danach
c) den Nebenmanipulator (4.4), der in (4.3.8) schwenkt, diesem Punkt entgegen verschieben muss, geführt durch das von diesem zum Körper des gemessenen Benutzers (8) hin ausgestrahlte Lichtbündel, wobei dieses als Anfangspunkt der Entfernung zu verstehen ist, wobei von diesem Drehpunkt aus in Bezug auf eine durch diesen hindurchgehende Waagerechte das Instrument in seiner oberen Bewegung den Winkel Alpha bildet und in der unteren Richtung den Winkel Beta bildet, hiernach
d) der folgende anthropometrische Punkt, genauso wie der erste, geortet werden muss,
e) der Hauptmanipulator (4.3) , genauso wie in b), dem folgenden anthropometrischen Punkt entgegen verschoben werden muss,
f) machen Sie das Maß in der schwenkbaren Voranzeige (4.3.8) sichtbar und geben Sie, mittels Druck des Steuergeräts (4.3.5), falls es erforderlich ist, die digitalen Daten ein,
i) setzen Sie, indem Sie zweimal das Steuergerät (4.3.5) drücken, den aufgezeichneten Wert zurück und löschen Sie ihn, so gibt es ein Verfahren, das durch die Vorrichtung erleichtert wird, die es ermöglicht, Messungen aufzuzeichnen, die entfernt an einem Körpersegment des gemessenen Benutzers (8) ausgerichtet werden, wobei die Entfernung und gleichzeitig die eingeschlossenen positiven und negativen Winkel zu der horizontalen Achse gebildet werden, die durch ihre Gelenkbasis hindurchgeht, wobei eine solche Lösung die instrumentelle, operative und gestische Stabilität sowie die Verringerung der dafür erforderlichen Zeit verbessert.

30. Systemisches Verfahren nach den vorhergehenden Ansprüchen, **dadurch gekennzeichnet, dass** es die folgenden Phasen umfasst:.
a) Transport der komplementären Einheiten (3 und 4) einzeln in Schutzvorrichtungen,
b) Entfalten der komplementären Einheiten (3 und 4),
c) Koppeln der komplementären Einheiten (3 und 4),
d) Anschließen von Gebergeräten für eine Informationsübertragung an ein Notebook oder dergleichen,
e) Nivellement der Einheit, unterstützt durch die in dem Notebook oder dergleichen installierte Software,
f) Verfahren des Lokalisierens und Aufzeichnens der Muster-, der vertikalen und horizontalen schrägen und der horizontalen ausgerichteten Messung,
g) Messung der drei durch den Haltungsausrichter (3) aufgenommenen Haltungen,
h) Messung in den vier Projektionsebenen durch die komplementären Einheiten (3 und 4), so gibt es ein systemisches Verfahren, das durch die Vorrichtung, die Messverfahren und die Software erleichtert wird, die es ermöglichen, einen Plan am Ort der Messung auszuführen, wobei die operativen Instanzen und die Verringerung der für dieselben erforderlichen Zeit erleichtert werden, wobei außerdem eine neue Weise des Messens des Körpers in einer, in Bezug auf den messenden Benutzer, instrumentell stabilen und, in Bezug auf den gemessenen Benutzer, nicht invasiven Form vorgeschlagen wird, die außerdem durch die digitale und automatische Leistungsfähigkeit, welche die Erfindung bereitstellt, erleichtert wird.
